(19) Europäisches Patentamt
European Patent Office
Office européen des brevets

(11) EP 0 694 544 A1

(12) **EUROPÄISCHE PATENTANMELDUNG**

(43) Veröffentlichungstag:
**31.01.1996 Patentblatt 1996/05**

(51) Int. Cl.$^6$: **C07D 413/04**, A61K 31/42

(21) Anmeldenummer: **95110628.5**

(22) Anmeldetag: **07.07.1995**

(84) Benannte Vertragsstaaten:
**AT BE CH DE DK ES FR GB GR IE IT LI LU MC NL PT SE**

(30) Priorität: **20.07.1994 DE 4425609**

(71) Anmelder: **BAYER AG**
**D-51368 Leverkusen (DE)**

(72) Erfinder:
- **Riedl, Bernd, Dr.**
  **D-42115 Wuppertal (DE)**
- **Häbich, Dieter, Dr.**
  **D-42115 Wuppertal (DE)**
- **Stolle, Andreas, Dr.**
  **D-42115 Wuppertal (DE)**
- **Wild, Hanno, Dr.**
  **Orange, Connecticut 06477 (US)**
- **Endermann, Rainer, Dr.**
  **D-42113 Wuppertal (DE)**
- **Bremm, Klaus Dieter, Dr.**
  **D-45661 Recklinghausen (DE)**
- **Kroll, Hein-Peter, Dr.**
  **D-42115 Wuppertal (DE)**
- **Labischinski, Harald, Prof. Dr.**
  **D-42109 Wuppertal (DE)**
- **Schaller, Klaus, Dr.**
  **D-42109 Wuppertal (DE)**
- **Werling , Hans-Otto, Dr.**
  **D-42113 Wuppertal (DE)**

(54) **Benzofuranyl- und Benzothienyloxazolidinone**

(57) Die vorliegende Erfindung betrifft Benzofuranyl- und Benzothienyloxazolidinone, Verfahren zu ihrer Herstellung und ihre Verwendung als Arzneimittel, insbesondere als antibakterielle Arzneimittel.

EP 0 694 544 A1

## Beschreibung

Die vorliegende Erfindung betrifft Benzofuranyl- und Benzothienyloxazolidinone, Verfahren zu ihrer Herstellung und ihre Verwendung als Arzneimittel, insbesondere als antibakterielle Arzneimittel.

Aus den Publikationen US 5 254 577, US 4 705 799, EP 311 090, US 4 801 600, US 4 921 869, US 4 965 268, EP 312 000 und C.H. Park et al., J. Med. Chem. 35, 1156 (1992) sind N-Aryloxazolidinone mit antibakterieller Wirkung bekannt.

Die vorliegende Erfindung betrifft Benzofuranyl- und Benzothienyloxazolidinone der allgemeinen Formel (I)

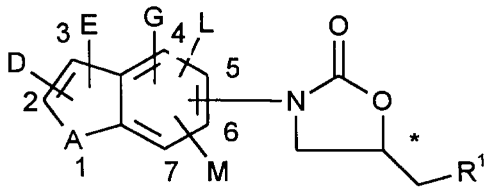

(I)

in welcher

| | |
|---|---|
| $R^1$ | für Azido, Hydroxy oder für eine Gruppe der Formel $-OR^2$, $-O-SO_2R^3$ oder $-NR^4R^5$ steht, worin |
| $R^2$ | geradkettiges oder verzweigtes Acyl mit bis zu 8 Kohlenstoffatomen oder eine Hydroxyschutzgruppe bedeutet, |
| $R^3$ | geradkettiges oder verzweigtes Alkyl mit bis zu 4 Kohlenstoffatomen oder Phenyl bedeutet, das gegebenenfalls durch geradkettiges oder verzweigtes Alkyl mit bis zu 4 Kohlenstoffatomen substituiert ist, |
| $R^4$ und $R^5$ | gleich oder verschieden sind und Cycloalkyl mit 3 bis 6 Kohlenstoffatomen, Wasserstoff, Phenyl oder geradkettiges oder verzweigtes Alkyl mit bis zu 8 Kohlenstoffatomen oder eine Aminoschutzgruppe bedeuten, oder |
| $R^4$ oder $R^5$ | eine Gruppe der Formel $-CO-R^6$ bedeutet, worin |
| $R^6$ | Cycloalkyl mit 3 bis 6 Kohlenstoffatomen, geradkettiges oder verzweigtes Alkyl oder Alkoxy mit jeweils bis zu 8 Kohlenstoffatomen, Phenyl oder Wasserstoff bedeutet, |
| A | für ein Sauerstoff- oder Schwefelatom steht, |
| D, E, G, L und M | gleich oder verschieden sind und für Wasserstoff, Carboxy, Halogen, Cyano, Mercapto, Formyl, Trifluormethyl, Nitro, geradkettiges oder verzweigtes Alkoxy, Alkoxycarbonyl, Alkylthio oder Acyl mit jeweils bis zu 6 Kohlenstoffatomen oder für geradkettiges oder verzweigtes Alkyl mit bis zu 6 Kohlenstoffatomen stehen, das seinerseits durch Hydroxy, durch geradkettiges oder verzweigtes Alkoxy oder Acyl mit bis zu 5 Kohlenstoffatomen durch einen Rest der Formel |

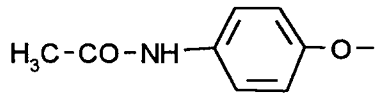,

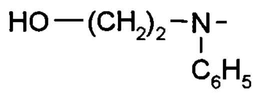,

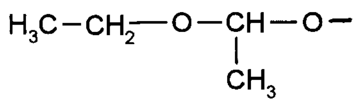

oder durch eine Gruppe der Formel $-NR^7R^8$ substituiert sein kann,
worin

$R^7$ und $R^8$ gleich oder verschieden sind und Wasserstoff, geradkettiges oder verzweigtes Alkyl mit bis zu 4 Kohlenstoffatomen oder Phenyl bedeuten, oder gemeinsam mit dem Stickstoffatom einen 5- bis 6-gliedrigen, gesättigten Heterocyclus mit gegebenenfalls einem weiteren Heteroatom aus der Serie N, S und/oder O bilden, der seinerseits, gegebenenfalls auch an einem weiteren Stickstoffatom, durch geradkettiges oder verzweigtes Alkyl oder Acyl mit bis zu 3 Kohlenstoffatomen substituiert sein kann,
und/oder
gegebenenfalls für eine Gruppe der Formel $-NR^{7'}R^{8'}$ stehen,
worin

$R^{7'}$ und $R^{8'}$ gleich oder verschieden sind und die oben angegebene Bedeutung von $R^7$ und $R^8$ haben und mit dieser gleich oder verschieden sind,
und/oder
gegebenenfalls für $(C_2$-$C_8)$-Alkenylphenyl, Phenyl oder durch einen 5- oder 6-gliedrigen gesättigten oder ungesättigten Heterocyclus mit bis zu 3 Heteroatomen aus der Reihe S, N und/oder O stehen, die ihrerseits gegebenenfalls durch eine Gruppe der Formel $-CO-NR^9R^{10}$, $-NR^{11}R^{12}$, $-NR^{13}-SO_2-R^{14}$, $-R^{15}R^{16}N-SO_2-$ oder $-R^{17}-S(O)_a-$ substituiert sind,
worin

a eine Zähl 0, 1 oder 2 bedeutet,

$R^9$, $R^{10}$, $R^{13}$, $R^{15}$ und $R^{16}$ gleich oder verschieden sind und Wasserstoff, geradkettiges oder verzweigtes Alkyl mit bis zu 6 Kohlenstoffatomen oder Phenyl bedeuten,

$R^{11}$ und $R^{12}$ gleich oder verschieden sind und die oben angegebene Bedeutung von $R^7$ und $R^8$ haben und mit dieser gleich oder verschieden sind,

$R^{14}$ und $R^{17}$ gleich oder verschieden sind und die oben angegebene Bedeutung von $R^3$ haben und mit dieser gleich oder verschieden sind,
und/oder ihrerseits gegebenenfalls bis zu 2-fach gleich oder verschieden durch Carboxy, Halogen, Cyano, Mercapto, Formyl, Trifluormethyl, Nitro, Phenyl, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy, durch geradkettiges oder verzweigtes Alkoxy oder Acyl mit bis zu 5 Kohlenstoffatomen oder durch eine Gruppe der Formel $-NR^{18}R^{19}$ substituiert sein kann,
worin

| | |
|---|---|
| $R^{18}$ und $R^{19}$ | die oben angegebene Bedeutung von $R^7$ und $R^8$ haben und mit dieser gleich oder verschieden sind, |

und deren Salze und S-Oxide.

Physiologisch unbedenkliche Salze der Benzofuranyl- und Benzothienyloxazolidinone können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabiethylamin, 1-Ephenamin oder Methyl-piperidin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren als auch Diastereomeren oder deren jeweiligen Mischungen. Die Racemformen lassen sich ebenso wie die Diastereomeren in bekannter Weise in die stereoisomer einheitlichen Bestandteile trennen.

Heterocyclus steht im allgemeinen für einen 5- bis 6-gliedrigen, gesättigten oder ungesättigten Ring, der als Heteroatome bis zu 3 Sauerstoff-, Schwefel- und/oder Stickstoffatome enthalten kann. Bevorzugt werden genannt: Thienyl, Furyl, Pyrrolyl, Pyrazolyl, Pyridyl, Pyrimidyl, Pyrazinyl, Pyridazinyl, Thiazolyl, Oxazolyl, Imidazolyl, Pyrrolidinyl, Piperidinyl oder Piperazinyl.

Dazu gehören auch über N-gebundene, 5- bis 6-gliedrige gesättigte Heterocyclen, die außerdem als Heteroatome bis zu 2 Sauerstoff-, Schwefel- und/oder Stickstoffatome enthalten können, wie beispielsweise Piperidyl, Morpholinyl oder Piperazinyl oder Pyrrolidinyl. Besonders bevorzugt sind Piperidyl und Pyrrolidinyl.

Hydroxyschutzgruppe im Rahmen der oben angegebenen Definition steht im allgemeinen für eine Schutzgruppe aus der Reihe: Trimethylsilyl, Triisopropylsilyl, tert.Butyl-dimethylsilyl, Benzyl, Benzyloxycarbonyl, 2-Nitrobenzyl, 4-Nitrobenzyl, tert. Butyloxycarbonyl, Allyloxycarbonyl, 4-Methoxybenzyl, 4-Methoxybenzyloxycarbonyl, Tetrahydropyranyl, Formyl, Acetyl, Trichloracetyl, 2,2,2-Trichlorethoxycarbonyl, Methoxyethoxymethyl, [2-(Trimethylsilyl)ethoxy]methyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Acetyl, tert. Butyldimethylsilyl oder Tetrahydropyranyl.

Aminoschutzgruppen im Rahmen der Erfindung sind die üblichen in der Peptid-Chemie verwendeten Aminoschutzgruppen.

Hierzu gehören bevorzugt: Benzyloxycarbonyl, 2,4-Dimethoxybenzyloxycarbonyl, 4-Methoxybenzyloxycarbonyl, Methoxycarbonyl, Ethoxycarbonyl, tert.Butoxycarbonyl, Allyloxycarbonyl, Phthaloyl, 2,2,2-Trichlorethoxycarbonyl, Fluorenyl-9-methoxycarbonyl, Formyl, Acetyl, 2-Chloracetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl, 4-Methoxyphenyl oder Triphenylmethyl.

Bevorzugt sind Verbindungen der allgemeinen Formel (I),
in welcher

| | |
|---|---|
| $R^1$ | für Azido, Hydroxy oder für eine Gruppe der Formel $-OR^2$, $-OSO_2R^3$ oder $-NR^4R^5$ steht,<br>worin |
| $R^2$ | geradkettiges oder verzweigtes Acyl mit bis zu 6 Kohlenstoffatomen oder Benzyl bedeutet, |
| $R^3$ | geradkettiges oder verzweigtes Alkyl mit bis zu 3 Kohlenstoffatomen, Phenyl oder Toluoyl bedeutet, |
| $R^4$ und $R^5$ | gleich oder verschieden sind und<br>Cyclopropyl, Cycylopentyl, Cyclohexyl, Wasserstoff, Phenyl oder geradkettiges oder verzweigtes Alkyl oder Alkoxy mit jeweils bis zu 6 Kohlenstoffatomen, tert. Butoxycarbonyl oder Benzyloxycarbonyl bedeuten,<br>oder |
| $R^4$ oder $R^5$ | eine Gruppe der Formel $-CO-R^6$ bedeutet,<br>worin |
| $R^6$ | Cyclopropyl, Cyclobutyl, Cyclopentyl, Cyclohexyl oder geradkettiges oder verzweigtes Alkyl oder Alkoxy mit jeweils bis zu 6 Kohlenstoffatomen, Phenyl oder Wasserstoff bedeutet, |
| A | für ein Sauerstoff- oder Schwefelatom steht, |

D, E, G, L und M gleich oder verschieden sind und für Wasserstoff, Carboxy, Fluor, Chlor, Brom, Jod, Cyano, Mercapto, Trifluormethyl, Formyl, Nitro, geradkettiges oder verzweigtes Alkoxy, Alkoxycarbonyl, Alkylthio oder Acyl mit jeweils bis zu 4 Kohlenstoffatomen oder für geradkettiges oder verzweigtes Alkyl mit bis zu 4 Kohlenstoffatomen stehen, das seinerseits durch Hydroxy, durch geradkettiges oder verzweigtes Alkoxy oder Acyl mit bis zu 4 Kohlenstoffatomen durch einen Rest der Formel

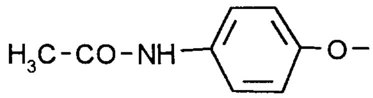 ,

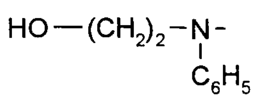 ,

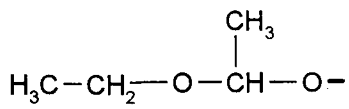

oder durch eine Gruppe der Formel $-NR^7R^8$ gegebenenfalls substituiert sein kann,
worin

$R^7$ und $R^8$ gleich oder verschieden sind und Wasserstoff, geradkettiges oder verzweigtes Alkyl mit bis zu 3 Kohlenstoffatomen oder Phenyl bedeuten,
oder gemeinsam mit dem Stickstoffatom einen Morpholinyl-, Pyrrolidinyl-, Piperazinyl- oder Piperidylring bilden, die gegebenenfalls, auch über die freie N-Funktion, durch Methyl, Ethyl oder Acetyl substituiert sind,
und/oder
gegebenenfalls für eine Gruppe der Formel
$-NR^{7'}R^{8'}$ stehen,
worin

$R^{7'}$ und $R^{8'}$ die oben angegebene Bedeutung von $R^7$ und $R^8$ haben und mit dieser gleich oder verschieden sind,
und/oder
gegebenenfalls für ($C_2$-$C_4$)-Alkenylphenyl, Phenyl, Pyridyl oder Thienyl stehen, die ihrerseits gegebenenfalls durch eine Gruppe der Formel $-CO-NR^9R^{10}$, $-NR^{11}R^{12}$, $-NR^{13}-SO_2-R^{14}$, $-R^{15}R^{16}N-SO_2-$ oder $-R^{17}-S(O)_a$-substituiert sind,
worin

a eine Zahl 0, 1 oder 2 bedeutet,

$R^9$, $R^{10}$, $R^{13}$, $R^{15}$ und $R^{16}$ gleich oder verschieden sind und Wasserstoff, geradkettiges oder verzweigtes Alkyl mit bis zu 4 Kohlenstoffatomen oder Phenyl bedeuten,

$R^{11}$ und $R^{12}$ gleich oder verschieden sind und die oben angegebene Bedeutung von $R^7$ und $R^8$ haben und mit dieser gleich oder verschieden sind,

$R^{14}$ und $R^{17}$ gleich oder verschieden sind und die oben angegebene Bedeutung von $R^3$ haben und mit dieser gleich oder verschieden sind,
und/oder ihrerseits gegebenenfalls bis zu 2-fach gleich oder verschieden durch Carboxy, Fluor, Chlor, Brom, Jod, Cyano, Mercapto, Trifluormethyl, Formyl, Nitro, Phenyl, geradkettiges oder verzweigtes Alkoxy, Alkoxycarbonyl, Alkylthio oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, durch geradkettiges oder verzweigtes Alkoxy oder Acyl mit bis zu 4 Kohlenstoffatomen oder durch eine

Gruppe der Formel $-NR^{18}R^{19}$ gegebenenfalls substituiert sein kann, worin

$R^{18}$ und $R^{19}$ die oben angegebene Bedeutung von $R^7$ und $R^8$ haben und mit dieser gleich oder verschieden sind,

und deren Salze und S-Oxide.

Besonders bevorzugt sind Verbindungen der allgemeinen Formel (I), in welcher

$R^1$ für Azido, Hydroxy oder für eine Gruppe der Formel $-OR^2$, $-OSO_2R^3$ oder $-NR^4R^5$ steht, worin

$R^2$ geradkettiges oder verzweigtes Acyl mit bis zu 6 Kohlenstoffatomen bedeutet,

$R^3$ Methyl, Ethyl, Phenyl oder Toluoyl bedeutet,

$R^4$ und $R^5$ gleich oder verschieden sind und Cyclopropyl, Cyclopentyl, Cyclohexyl, Wasserstoff, Phenyl oder geradkettiges oder verzweigtes Alkyl mit bis zu 5 Kohlenstoffatomen bedeuten, oder

$R^4$ oder $R^5$ eine Gruppe der Formel $-CO-R^6$ bedeutet, worin

$R^6$ Cyclopropyl, Cyclopentyl, Cyclohexyl oder geradkettiges oder verzweigtes Alkyl oder Alkoxy mit jeweils bis zu 4 Kohlenstoffatomen, Wasserstoff oder Phenyl bedeutet,

A für ein Sauerstoff- oder Schwefelatom steht,

D, E, G, L und M gleich oder verschieden sind und für Wasserstoff, Carboxy, Fluor, Chlor, Brom, Jod, Cyano, Formyl, Trifluormethyl, Nitro, geradkettiges oder verzweigtes Alkoxy, Alkoxycarbonyl oder Acyl mit jeweils bis zu 4 Kohlenstoffatomen oder für geradkettiges oder verzweigtes Alkyl mit bis zu 4 Kohlenstoffatomen stehen, das seinerseits durch Hydroxy, durch geradkettiges oder verzweigtes Alkoxy oder Acyl mit bis zu 4 Kohlenstoffatomen, durch einen Rest der Formel

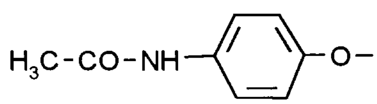
,

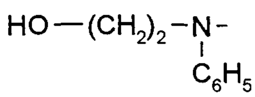
oder

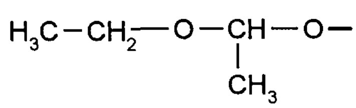

oder durch eine Gruppe der Formel $-NR^7R^8$ gegebenenfalls substituiert sein kann, worin

$R^7$ und $R^8$ gleich oder verschieden sind und Wasserstoff oder Methyl bedeuten, oder gemeinsam mit dem Stickstoffatom einen Morpholinyl-, Pyrrolidinyl-, Piperazinyl- oder Piperidylring bilden, die gegebenenfalls, auch über die freie N-Funktion, durch Methyl, Ethyl oder Acetyl substituiert sind,

und/oder

gegebenenfalls für eine Gruppe der Formel $-NR^{7'}R^{8'}$ stehen, worin

$R^{7'}$ und $R^{8'}$ die oben angegebene Bedeutung von $R^7$ und $R^8$ haben und mit dieser gleich oder verschieden sind, und/oder

gegebenenfalls für 2-Phenylvinyl, Phenyl, Pyridyl oder Thienyl stehen, die ihrerseits gegebenenfalls durch eine Gruppe der Formel $-CO-NR^9R^{10}$ oder $-NR^{11}R^{12}$ substituiert sind,
worin

$R^9$ und $R^{10}$ gleich oder verschieden sind und Wasserstoff oder Methyl bedeuten,

$R^{11}$ und $R^{12}$ gleich oder verschieden sind und die oben angegebene Bedeutung von $R^7$ und $R^8$ haben und mit dieser gleich oder verschieden sind,
und/oder ihrerseits gegebenenfalls bis zu 2-fach gleich oder verschieden durch Carboxy, Fluor, Chlor, Brom, Jod, Cyano, Formyl, Trifluormethyl, Nitro, Phenyl, geradkettiges oder verzweigtes Alkoxy, Alkoxycarbonyl oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, durch geradkettiges oder verzweigtes Alkoxy oder Acyl mit bis zu 4 Kohlenstoffatomen oder durch eine Gruppe der Formel $-NR^{18}R^{19}$ gegebenenfalls substituiert sein kann,
worin

$R^{18}$ und $R^{19}$ die oben angegebene Bedeutung von $R^7$ und $R^8$ haben und mit dieser gleich oder verschieden sind,
und deren Salze und S-Oxide.

Ganz besonders bevorzugt sind Verbindungen der allgemeinen Formel (I),
in welcher
G, L und M für Wasserstoff stehen und der Oxazolidinonrest in der Position 5 oder 6 an den Phenylring angebunden ist.

Außerdem wurden Verfahren zur Herstellung der erfindungsgemäßen Verbindungen der allgemeinen Formel (I) gefunden, dadurch gekennzeichnet, daß man

[A] Verbindungen der allgemeinen Formeln (II) oder (III)

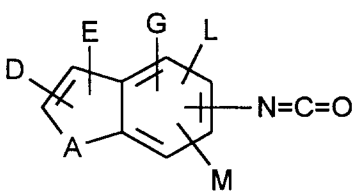 (II) oder 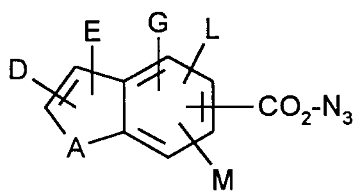 (III)

in welchen
A,D, E, G, L und M die oben angegebene Bedeutungen haben,
mit Lithiumbromid/$(C_4H_9)_3$ P(O) und Epoxiden der allgemeinen Formel (IV)

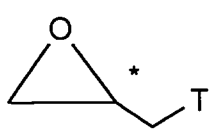 (IV)

in welcher

T für $C_1$-$C_6$-Acyloxy steht,

in inerten Lösemitteln, gegebenenfalls in Anwesenheit einer Base
umsetzt,
und im Fall $R^1$ = OH durch eine typische Esterverseifung oder durch eine typische Umesterung die Hydroxyfunktion freisetzt,
oder

[B] Verbindungen der allgemeinen Formel (V)

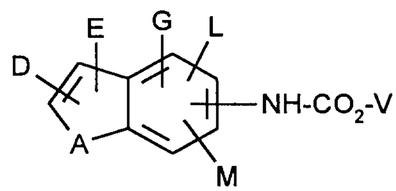

(V)

in welcher
A, D, E, G, L und M die oben angegebene Bedeutung haben
und

V für eine typische Schutzgruppe, vorzugsweise Benzyl steht,

in inerten Lösemitteln und in Anwesenheit einer Base, beispielsweise Lithiumalkylen oder Lithium-N-alkyl- oder Lithium-N-silylalkylamiden, vorzugsweise N-Butyllithium, mit Epoxiden der allgemeinen Formel (IV) umsetzt,
oder

[C] im Fall $R^1$ = OH, zunächst Verbindungen der allgemeinen Formel (III) durch Abspaltung von Stickstoff in Alkoholen in die Verbindungen der allgemeinen Formel (Va)

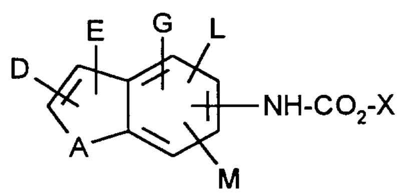

(Va)

in welcher
A, D, E, G, L und M die oben angegebene Bedeutung haben
und

X für geradkettiges oder verzweigtes $C_2$-$C_6$-Alkyl, vorzugsweise n-Butyl steht,

überführt,
und in einem zweiten Schritt wie unter [A] beschrieben in inerten Lösemitteln und in Anwesenheit einer Base, vorzugsweise Lithium-N-alkyl-oder N-Silylalkylamiden oder n-Butyllithium und Epoxiden der allgemeinen Formel (IV) umsetzt,
oder

[D] Verbindungen der allgemeinen Formel (VI)

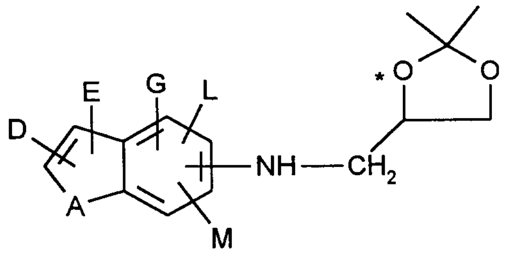

(VI)

in welcher
A, D, E, G, L und M die oben angegebene Bedeutung haben,
entweder direkt mit Säuren und Kohlensäurediethylester
umsetzt,
oder zunächst durch Umsetzung der Verbindungen der allgemeinen Formel (VI) mit Säuren die Verbindungen der allgemeinen Formel (VII)

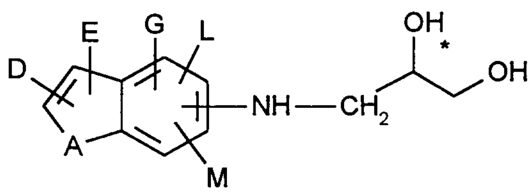

(VII)

in welcher
A, D, E, G, L und M die oben angegebene Bedeutung haben
herstellt,
und anschließend in Anwesenheit eines Hilfsmittels in inerten Lösemitteln cyclisiert,
oder

[E] zunächst Verbindungen der allgemeinen Formel (Ia)

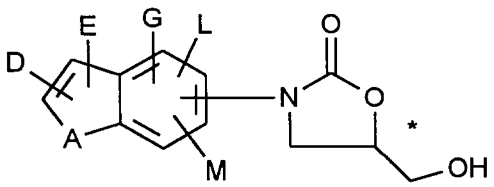

(Ia)

in welcher
A, D, E, G, L und M die oben angegebene Bedeutung haben,
durch Umsetzung mit $(C_1-C_4)$-Alkyl- oder Phenylsulfonsäurechloriden in inerten Lösemitteln und in Anwesenheit einer

Base in die entsprechenden Verbindungen der allgemeinen Formel (Ib)

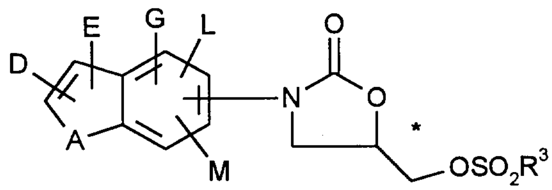

(Ib)

in welcher
A, D, E, G, L, M und $R^3$ die oben angegebene Bedeutung haben,
überführt,
anschließend mit Natriumazid in inerten Lösemitteln die Azide der allgemeinen Formel (Ic)

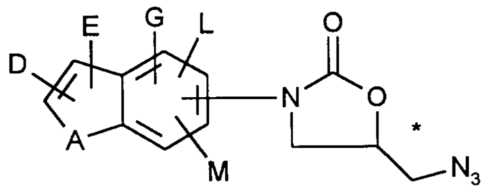

(Ic)

in welcher
A, D, E, G, L und M die oben angegebene Bedeutung haben,
herstellt,
in einem weiteren Schritt durch Umsetzung mit $(C_1\text{-}C_4\text{-}O)_3\text{-}P$ oder $Ph_3P$, vorzugsweise $(CH_3O)_3P$ in inerten Lösemitteln und mit Säuren in die Amine der allgemeinen Formel (Id)

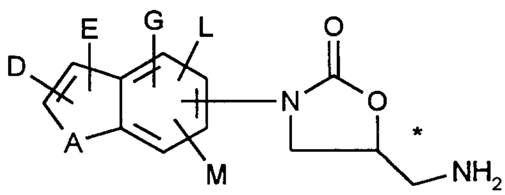

(Id)

in welcher
A, D, E, G, L und M die oben angegebene Bedeutung haben,
überführt,
und durch Umsetzung mit Acetanhydrid oder anderen Acylierungsmitteln der allgemeinen Formel (VIII)

$$Y\text{-}CO\text{-}R^6 \qquad \text{(VIII)}$$

in welcher
$R^6$ die oben angegebene Bedeutung hat
und

Y für Halogen, vorzugsweise für Chlor oder für den Rest $-OCOR^6$ steht,

in inerten Lösemitteln die Verbindungen der allgemeinen Formel (Ie)

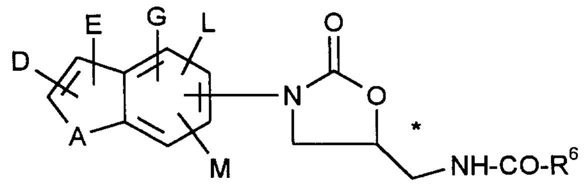

(Ie)

in welcher
A, D, E, G, L, M und $R^6$ die oben angegebene Bedeutung haben
herstellt,
oder

[F] Verbindungen der allgemeinen Formel (Ie) durch eine Halogenierung, gegebenenfalls in Anwesenheit eines Silberkatalysators, in die Verbindungen der allgemeinen Formel (If)

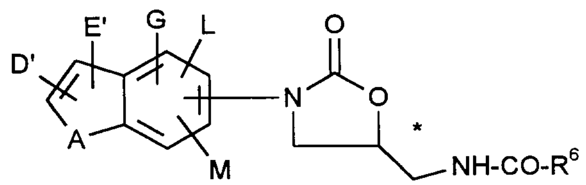

(If)

in welcher
D' oder E' für Halogen, vorzugsweise für Brom oder Jod steht,
und
A, G, L, M und $R^6$ die oben angegebene Bedeutung haben,
überführt,
oder

[G] Verbindungen der allgemeinen Formel (If) mit Verbindungen der allgemeinen Formel (IX)

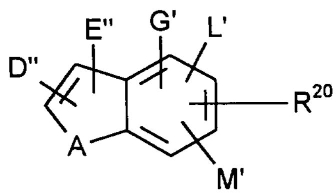

(IX)

in welcher

| | |
|---|---|
| A | die oben angegebene Bedeutung hat<br>und<br>D", E", G', L' und/oder M' einen der unter D, E, G, L und M oben aufgeführten, gegebenenfalls substituierten, monocyclischen Heterocyclen, Phenyl oder ($C_2$-$C_8$)-Alkenylphenyl stehen,<br>und |
| $R^{20}$ | für den Boronsäurerest $-B(OH)_2$ oder für einen zinnorganischen Rest der Formel $-SnR^{21}R^{22}R^{23}$ steht,<br>worin |

$R^{21}$, $R^{22}$ und $R^{23}$ gleich oder verschieden sind und $C_1$-$C_4$-Alkyl bedeuten,

in inerten Lösemitteln und in Anwesenheit eines Palladiumkatalysators umsetzt,
oder

[H] im Fall A=O
Verbindungen der Formel (X)

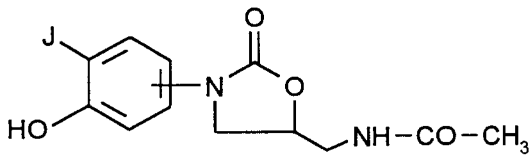

(X)

durch Umsetzung mit Propargylalkohol in inerten Lösemitteln und in dem System $Pd(P(C_6H_5)_3)_2(OAc)_2$/CuJ, NaOAc cyclisiert,
und im Fall der S-Oxide eine Oxidation durchführt,
und im Fall $R^4$, $R^5$, $R^9$, $R^{10}$, $R^{11}$, $R^{12}$, $R^{13}$, $R^{15}$, $R^{16}$, $R^{18}$ und $R^{19} \neq H$ eine Alkylierung nach üblichen Methoden durchführt,
und gegebenenfalls weitere Substituenten oder bereits vorhandene funktionelle Gruppen nach üblichen Methoden, wie beispielsweise Redoxreaktionen, Substitutionsreaktionen und/oder Verseifungen oder Ein- und Abbau von Schutzgruppen, einführt bzw. derivatisiert.

Die erfindungsgemäßen Verfahren können durch folgende Formelschemata beispielhaft erläutert werden:

[A]

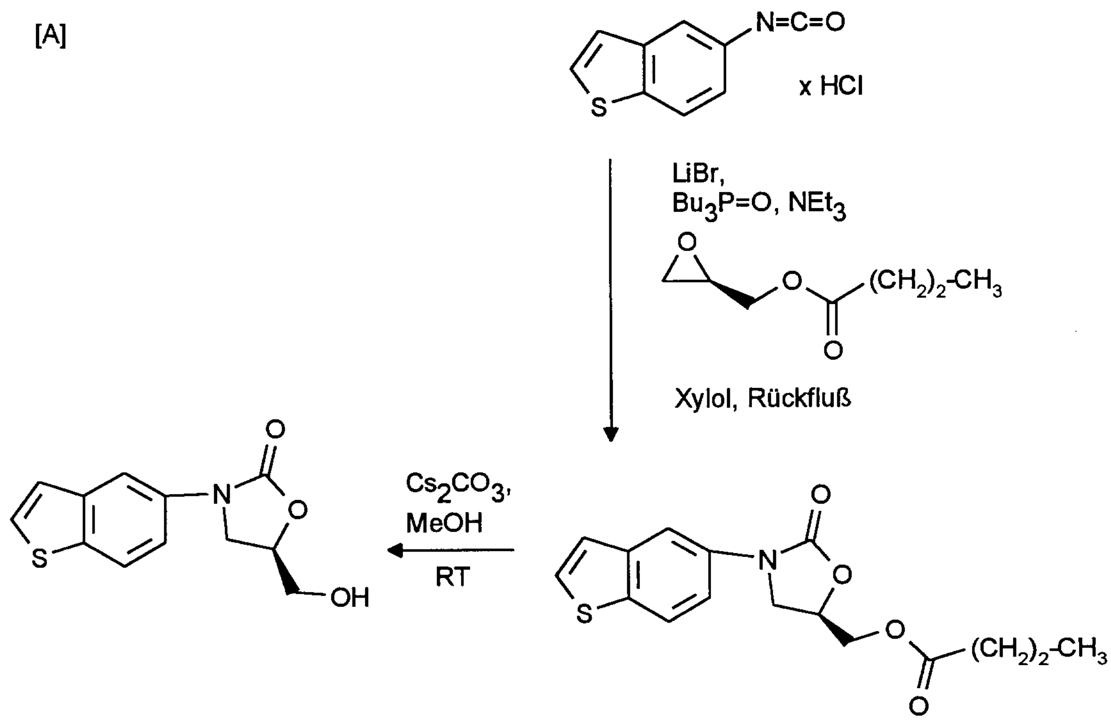

[A]

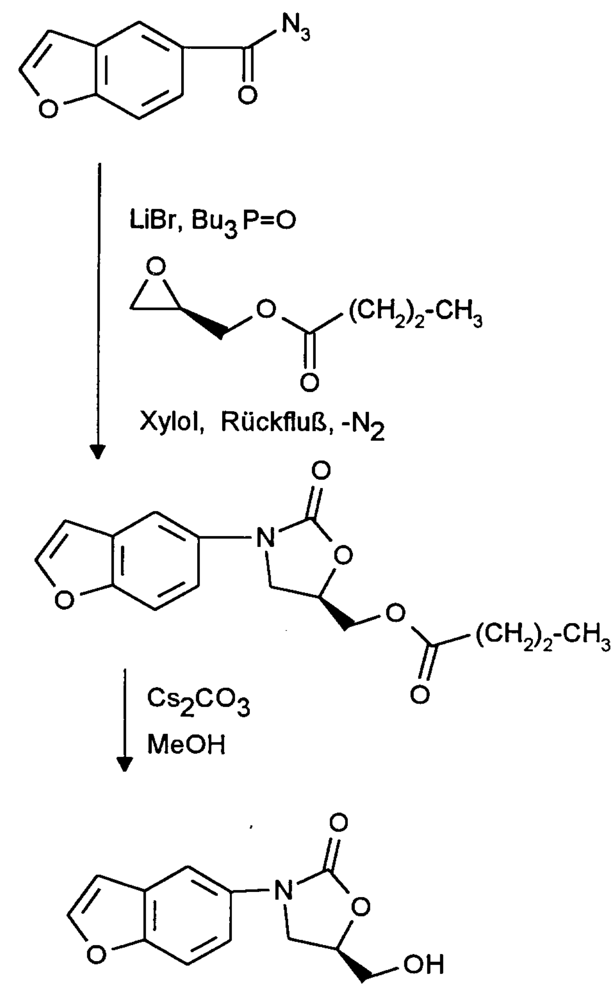
LiBr, $Bu_3P{=}O$
Xylol, Rückfluß, $-N_2$
$Cs_2CO_3$
MeOH

[B]

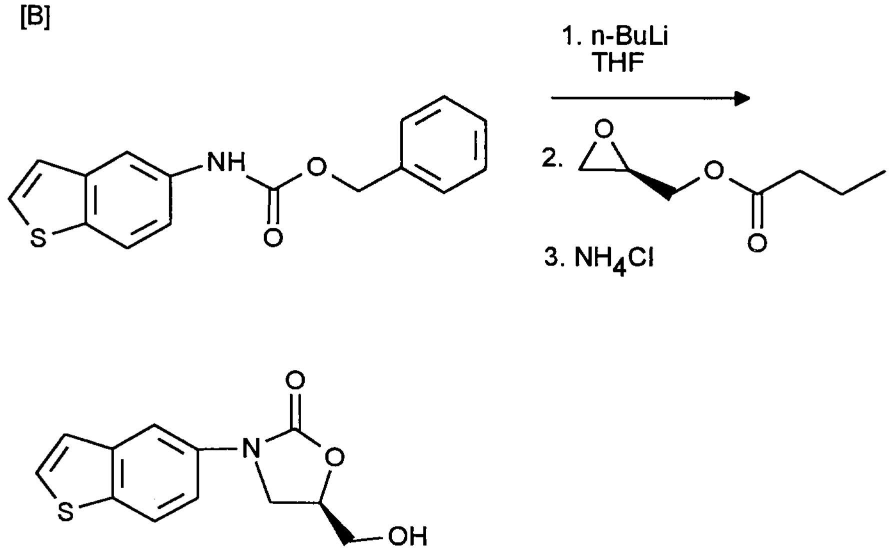
1. n-BuLi
THF
2.
3. $NH_4Cl$

[C]

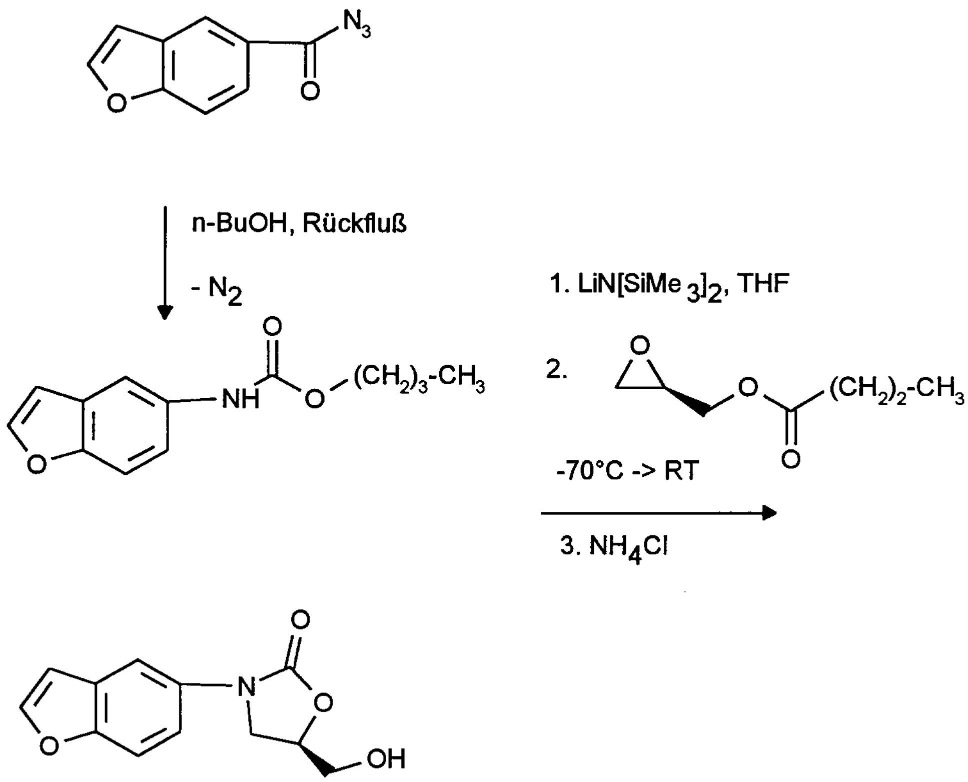

n-BuOH, Rückfluß
- $N_2$
1. $LiN[SiMe_3]_2$, THF
2.
$(CH_2)_2$-$CH_3$
$(CH_2)_3$-$CH_3$
-70°C -> RT
3. $NH_4Cl$


[D]

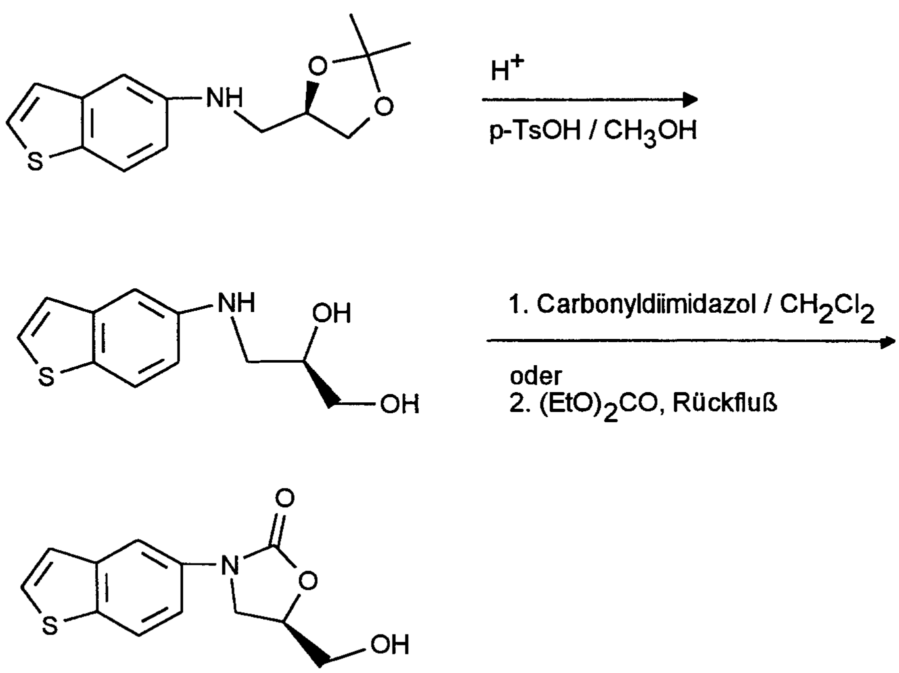

$H^+$
p-TsOH / $CH_3OH$
1. Carbonyldiimidazol / $CH_2Cl_2$
oder
2. $(EtO)_2CO$, Rückfluß

[E]

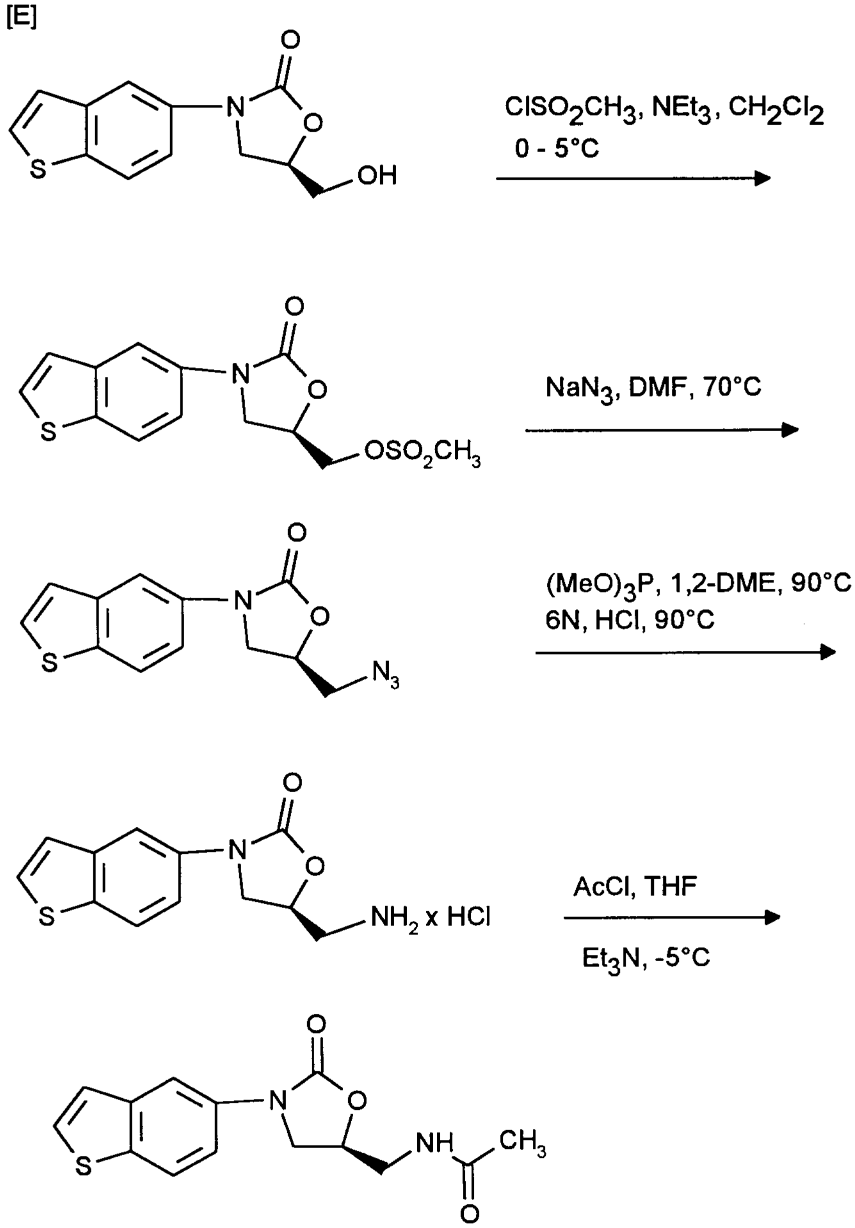
ClSO2CH3, NEt3, CH2Cl2
0 - 5°C
NaN3, DMF, 70°C
(MeO)3P, 1,2-DME, 90°C
6N, HCl, 90°C
x HCl
AcCl, THF
Et3N, -5°C

[F]

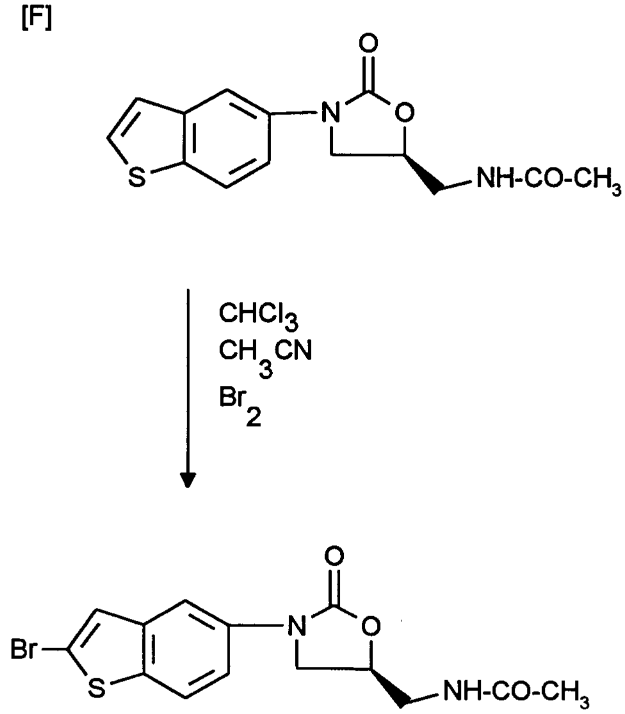


[G]

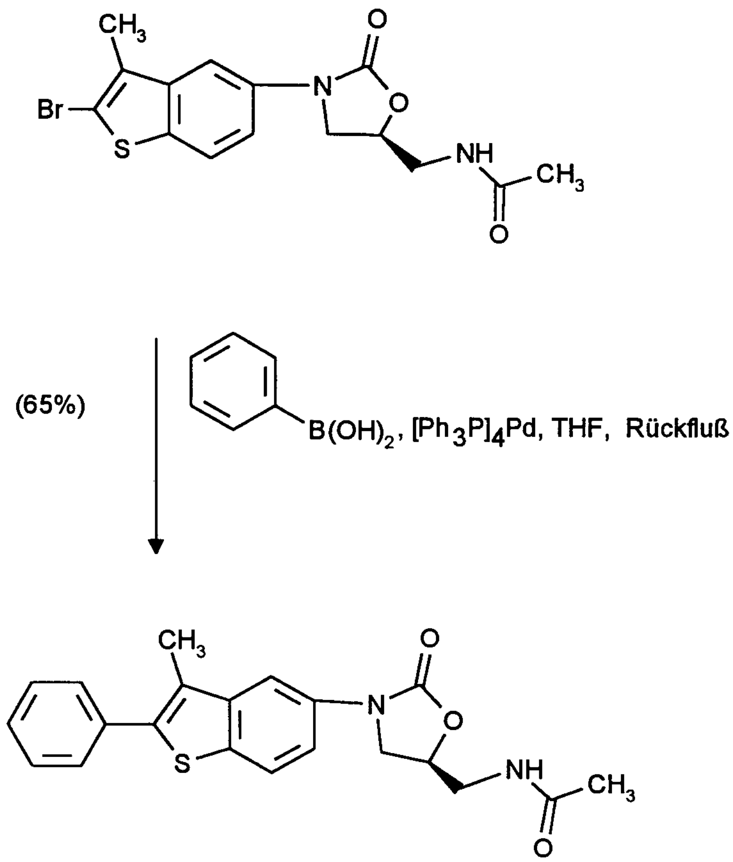

[H]

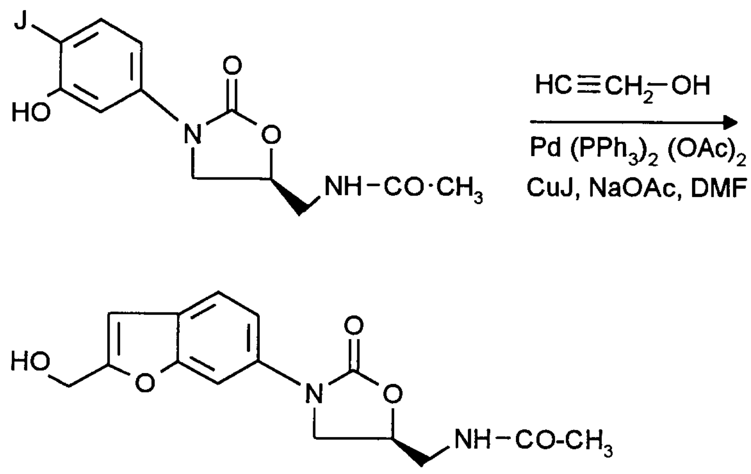


Als Lösemittel eignen sich in Abhängigkeit von den einzelnen Verfahrensschritten die üblichen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, 1,2-Dimethoxyethan, Tetrahydrofuran, Glykoldimethylether oder tert.Butylmethylether, oder Ketone wie Aceton oder Butanon, oder Amide wie Dimethylformamid oder Hexamethyl-phosphorsäuretriamid, oder Kohlenwasserstoffe wie Hexan, Benzol, Dichlorbenzol, Xylol oder Toluol, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden.

Als Basen eignen sich in Abhängigkeit von den einzelnen Verfahrensschritten die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Ethyldiisopropylamin, Triethylamin, Picolin, Pyridin oder N-Methylpiperidin, oder Amide wie Natriumamid oder Lithiumdiisopropylamid, oder Lithium-N-silylalkylamide, wie beispielsweise Lithium-N-(bis)triphenysilylamid oder Lithiumalkyle wie n-Butyllithium.

Die Base wird in einer Menge von 1 mol bis 10 mol, bevorzugt von 1 mol bis 3 mol bezogen auf 1 mol der Verbindungen der allgemeinen Formeln (II), (III), (IV) und (Va) eingesetzt.

Alle Umsetzungen werden im allgemeinen bei normalem, erhöhtem oder bei erniedrigtem Druck durchgeführt (z.B. 0,5 bis 5 bar). Im allgemeinen arbeitet man bei Normaldruck.

Das Verfahren [A] erfolgt bevorzugt in Xylol oder Dichlorbenzol, gegebenenfalls in Gegenwart von Triethylamin unter Rückfluß.

Die basenkatalysierte Umesterung wird mit einem der oben aufgeführten Alkohole, vorzugsweise Methanol, in einem Temperaturbereich von -10°C bis +40°C, vorzugsweise bei Raumtemperatur durchgeführt.

Als Basen eignen sich im allgemeinen Natriumhydrogencarbonat, Natriummethanolat, Hydrazinhydrat, Kaliumcarbonat oder Caesiumcarbonat. Bevorzugt ist Caesiumcarbonat.

Das Verfahren [B] erfolgt in einem der oben aufgeführten Ether mit Lithiumalkylverbindungen oder Lithium-N-silylamiden, wie beispielsweise n-Butyllithium, Lithiumdiisopropylamid oder Lithium-bistrimethylsilylamid, vorzugsweise in Tetrahydrofuran und Lithium-bis-trimethylsilylamid oder n-Butyllithium, in einem Temperaturbereich von -100°C bis +20°C, vorzugsweise von -75°C bis -40°C.

Für das Verfahren [C] eignen sich für den 1. Schritt vorzugsweise die oben aufgeführten Alkohole, im Falle der anschließenden Cyclisierung Tetrahydrofuran.

Als Basen für die Cyclisierung eignen sich vorzugsweise die oben aufgeführten Lithium-N-silylalkylverbindungen oder n-Butyllithium. Besonders bevorzugt ist n-Butyllithium.

Der erste Reaktionsschritt wird bei der Siedetemperatur des entsprechenden Alkohols, die Cyclisierung in einem Temperaturbereich von -70°C bis Raumtemperatur durchgeführt.

Die Cyclisierung [D] wird in Anwesenheit eines Hilfsmittels und/oder in Anwesenheit einer Säure durchgeführt.

Als Säuren eignen sich im allgemeinen anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie

beispielsweise Essigsäure, Trifluoressigsäure, Trichloressigsäure oder Propionsäure, oder Sulfonsäuren mit $C_1$-$C_4$-Alkylresten oder Arylresten wie beispielsweise Methansulfonsäure, Ethansulfonsäure, Benzolsulfonsäure oder Toluolsulfonsäure. Besonders bevorzugt ist Salzsäure.

Die Säure wird in einer Menge von 1 mol bis 10 mol, bevorzugt von 1 mol bis 2 mol, bezogen auf 1 mol der Verbindungen der allgemeinen Formel (VI) eingesetzt.

Als Hilfsmittel eignen sich die üblichen Reagenzien wie Phosgen, Carbonyldiimidazol oder Kohlensäurediethylester oder Chlorameisensäuretrichlormethylester. Bevorzugt sind Carbonyldiimidazol, Kohlensäurediethylester oder Chlorameisensäuretrichlormethylester.

Als Lösemittel eignen sich die oben aufgeführten Halogenkohlenwasserstoffe. Bevorzugt ist Methylenchlorid.

Die Cyclisierungen erfolgen im allgemeinen in einem Temperaturbereich von -20°C bis 100°C, vorzugsweise bei -20°C bis Raumtemperatur.

Die Acylierung [E] erfolgt im allgemeinen in einem der oben aufgeführten Ether oder Halogenkohlenwasserstoffen, vorzugsweise Tetrahydrofuran oder Methylenchlorid, in einem Temperaturbereich von -30°C bis 50°C, bevorzugt von -10°C bis Raumtemperatur.

Die Kupplungsreaktion [G] mit den Boronsäure- und Zinnarylverbindungen erfolgt ebenfalls in einem der oben aufgeführten Ether oder Kohlenwasserstoffe, vorzugsweise Tetrahydrofuran oder Toluol und in Anwesenheit eines Palladiumkomplexes.

Als Palladiumkomplexe eignen sich beispielsweise $Pd[P(C_6H_5)_3]_4$, $[(C_6H_5)_3P]_2PdCl_2$ oder $(C_6H_5CN)_2PdCl_2$. Bevorzugt ist $[(C_6H_5)_3P]_4Pd$.

Die Umsetzung erfolgt in einem Temperaturbereich von Raumtemperatur bis 150°C, vorzugsweise bei der Siedetemperatur des jeweiligen Lösemittels.

Die Reduktionen erfolgen im allgemeinen mit Hydriden in inerten Lösemitteln oder mit Boranen, Diboranen oder ihren Komplexverbindungen.

Bevorzugt werden die Reduktionen mit Hydriden, wie komplexen Borhydriden oder Aluminiumhydriden sowie Boranen durchgeführt. Besonders bevorzugt werden hierbei Natriumborhydrid, Lithiumborhydrid, Natriumcyanoborhydrid, Lithiumaluminiumhydrid, Natrium-bis-(2-methoxyethoxy)aluminiumhydrid oder Boran-Tetrahydrofuran eingesetzt.

Die Reduktion erfolgt im allgemeinen in einem Temperaturbereich von -50°C bis zum jeweiligen Siedepunkt des Lösemittels, bevorzugt von -20°C bis +90°C.

Die Reduktionen können im allgemeinen durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder mit Hydriden oder Boranen in inerten Lösemitteln, gegebenenfalls in Anwesenheit eines Katalysators durchgeführt werden.

Bevorzugt wird die Reaktion mit Hydriden, wie komplexen Borhydriden oder Aluminiumhydriden durchgeführt. Besonders bevorzugt werden hierbei Natriumborhydrid, Lithiumaluminiumhydrid oder Natriumcyanoborhydrid eingesetzt.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden.

Die Oxidation zum N-oxid erfolgt im allgemeinen in einem der oben aufgeführten Lösemittel, vorzugsweise in Methylenchlorid mit Oxidationsmitteln wie beispielsweise Metachlorperbenzoesäure, Wasserstoffperoxid oder Peressigsäure, vorzugsweise mit Metachlorperbenzoesäure in einem Temperaturbereich von 0°C bis 80°C, bevorzugt von 0°C bis 40°C.

Die Abspaltung der Hydroxyschutzgruppen erfolgt im allgemeinen nach üblicher Methode, beispielsweise durch hydrogenolytische Spaltung der Benzylether in den oben aufgeführten inerten Lösemitteln in Anwesenheit eines Katalysators mit Wasserstoff-Gas.

Die Abspaltung der Aminoschutzgruppe erfolgt im allgemeinen, ebenfalls nach üblichen Methoden, und zwar vorzugsweise Boc mit Salzsäure in Dioxan, Fmoc mit Piperidin und Z mit HBr/HOAc oder durch Hydrogenolyse.

Die oben aufgeführten anderen Derivatisierungsreaktionen erfolgen im allgemeinen nach den in Compendium of Organic Synthetic Methods, T.T. Harrison und S. Harrison, Wiley Interscience, publizierten Methoden.

Bevorzugt werden Redoxreaktionen, reduktive Aminierung, Umesterung und die Halogenisierung von Methylgruppen mit N-Bromsuccinimid (NBS) oder N-Chlorsuccinimid (NCS) aufgeführt, die im folgenden beispielhaft erläutert werden.

Als Lösemittel für die Alkylierung eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Acetonitril, Aceton oder Nitromethan.

Ebenso ist es möglich, Gemische der genannten Lösemittel zu verwenden. Bevorzugt sind Dichlormethan, Dimethylsulfoxid und Dimethylformamid.

Die Alkylierung wird in den oben aufgeführten Lösemitteln bei Temperaturen von 0°C bis +150°C, vorzugsweise bei Raumtemperaturen bis +100°C, bei Normaldruck durchgeführt.

Die Amidierung und die Sulfoamidierung erfolgen im allgemeinen in inerten Lösemitteln in Anwesenheit einer Base und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwassserstoffe wie Benzol, Xylol, Toluol, Hexan, Cyclohexan, oder Erdölfraktionen, Nitromethan, Dimethylformamid, Acetonitril oder Tetrahydrofuran. Ebenso ist es möglich, Gemische der Lösemittel einzusetzen. Besonders bevorzugt sind Dichlormethan und Tetrahydrofuran.

Als Basen für die Amidierung und die Sulfoamidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.buylat, oder organische Amine wie Benzyltrimethylammomumhydroxid, Tetrabutylammoniumhydroxid, Pyridin, Triethylamin oder N-Methylpiperidin.

Die Amidierung und die Sulfoamidierung werden im allgemeinen in einem Temperaturbereich von 0°C bis 150°C, bevorzugt bei 25°C bis 40°C, durchgeführt.

Die Amidierung und die Sulfoamidierung werden im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Bei der Durchführung der Amidierung und der Sulfoamidierung wird die Base im allgemeinen in einer Menge von 1 bis 3 Mol, bevorzugt von 1 bis 1,5 Mol, bezogen auf 1 Mol der jeweiligen Carbonsäure, eingesetzt.

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphorsäurediphenylesteramid oder Methansulfonsäurechlorid, gegebenenfalls in Anwesenheit von Basen wie Triethylamin oder N-Ethylmorpholin oder N-Methylpiperidin oder 4-Dimethylaminopyridin.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol bezogen auf 1 Mol des Esters eingesetzt. Besonders bevorzugt verwendet man molare Mengen der Reaktanden.

Die Veresterung erfolgt im allgemeinen mit den entsprechenden Alkoholen in Anwesenheit von Säuren, vorzugsweise Schwefelsäure, in einem Temperaturbereich von 0°C bis 150°C, vorzugsweise von 50°C bis 100°C und Normaldruck.

Die Verbindungen der allgemeinen Formeln (IV), (VIII) und (IX) sind bekannt oder können nach üblichen Methoden hergestellt werden.

Die Verbindungen der allgemeinen Formel (VII) sind größtenteils neu und können beispielsweise wie oben beschrieben hergestellt werden.

Die Verbindungen der allgemeinen Formel (II) sind teilweise bekannt oder neu und können dann beispielsweise hergestellt werden, indem man die entsprechenden Amine mit Chlorameisensäuretrichlorethylester in einem der oben aufgeführten Lösemittel, vorzugsweise Xylol bei Rückflußtemperatur umsetzt.

Die Verbindungen der allgemeinen Formel (III) sind teilweise bekannt oder neu und können dann beispielsweise hergestellt werden, indem man ausgehend von den entsprechenen Carbonsäuren entweder mit Chlorameisensäureisobutylester / Aceton, Natriumazid/Wasser oder mit Diphenylphosphorylazid / Tetrahydrofuran oder mit Xylol oder

Methylenchlorid in Gegenwart einer der oben angegebenen Basen, vorzugsweise Triethylamin, bei -10°C bis Raumtemperatur umsetzt.

Die Verbindungen der allgemeinen Formeln (V) und (Va) sind teilweise bekannt oder neu und können entweder durch Abspaltung von Stickstoff aus den entsprechenden Carbonsäureaziden und Umsetzung mit den entsprechenden Alkoholen oder durch Umsetzung der entsprechenden Amine mit Chlorameisensäureestern, vorzugsweise Chlorameisensäurebenzylester in einem der oben aufgeführten Lösemittel, vorzugsweise Tetrahydrofuran oder Dioxan, in einem Temperaturbereich von -10°C bis 200°C, vorzugsweise von 0°C bis 150°C, hergestellt werden.

Die Verbindungen der allgemeinen Formel (VII) sind größtenteils neu und können wie oben beschrieben hergestellt werden.

Die Verbindungen der allgemeinen Formel (Ia) sind neu und können beispielsweise wie unter [A], [B], [D] oder [E] beschrieben hergestellt werden.

Die Verbindungen der allgemeinen Formeln (Ib), (Ic), (Id), (Ie) und (If) sind neu und können wie oben beschrieben hergestellt werden.

Die Verbindungen der allgemeinen Formel (VI) sind größtenteils bekannt oder neu und können beispielsweise hergestellt werden, indem man ausgehend von den freien Aminen (Ia) entweder mit dem Acetonid von Glycerinaldehyd in Methanol und in Anwesenheit von Natriumacetat / Natriumcyanborhydrid oder von Natriumboranat und Methanol in einem Temperaturbereich von -20°C bis +40°C, bevorzugt von -10°C bis 20°C und Normaldruck umsetzt.

Die Einführung des Halogenatoms Y (Verbindungen der allgemeinen Formel (If)) erfolgt im Fall von Brom und Jod entweder mit elementarem Brom oder Jod, oder im Fall von Brom oder Jod in Anwesenheit eines Silber-Salzes, in einem der oben aufgeführten Lösemittel, vorzugsweise Methylenchlorid, Acetonitril oder Chloroform, in einem Temperaturbereich von -30°C bis +60°C, bevorzugt von 0°C bis +30°C und Normaldruck.

Als Silber-Salze eignen sich beispielsweise Silbertetrafluoroborat, Silbertrifluormethansulfonat oder Silbertrifluoracetat.

Die Verbindung der Formel (X) wird vom Bedeutungsumfang der Publikation umfaßt, ist als Species aber neu und kann hergestellt werden, indem man ausgehend von dem bekannten 3-Methoxy-substituiertem Phenyl-2-oxazolidinon eine Jodierung mit $J_2$ in Anwesenheit von Ag(OAc) in Acetonitril und Chloroform durchführt und anschließend mit $BBr_3$ in Dichlormethan die Hydroxyfunktion freisetzt.

Das Verfahren [H] erfolgt in einem der oben aufgeführten Lösemittel, vorzugsweise Dimethylformamid in einem Temperaturbereich von 40°C bis 80°C, vorzugsweise 60°C und Normaldruck.

Die minimalen Hemmkonzentrationen (MHK) wurden per Reihenverdünnungsverfahren auf Iso-Sensitest Agar (Oxoid) bestimmt. Für jede Prüfungssubstanz wurde eine Reihe von Agarplatten hergestellt, die bei jeweils doppelter Verdünnung abfallende Konzentration des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. $10^4$ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

Die MHK-Werte wurden mit Hilfe der Mikrodilutionsmethode in BH-Medium bestimmt. Jede Prüfsubstanz wurde im Nährmedium gelöst. In der Mikrotiterplatte wurde durch serielle Verdünnung eine Konzentrationsreihe der Prüfsubstanzen angelegt. Zur Inokulation wurden Übernachtkulturen der Erreger verwandt, die zuvor im Nährmedium 1:250 verdünnt wurden. Zu 100 µl der verdünnten, wirkstoffhaltigen Nährlösungen wurden je 100 µl Inokulationslösung gegeben. Die Mikrotiterplatten wurden bei 37°C bebrütet und nach ca. 20 Stunden abgelesen. Der MHK-Wert (µg/ml) gibt die niedrigste

Wirkstoffkonzentration an, bei der kein Wachstum zu erkennen war.

MHK-Werte (µg/ml):

| Bsp.-Nr. | Staph. 133 | Staph. 48N | Staph 25701 | Staph. 9TV | E. coli Neumann | Klebs. 57 USA | Psdm. Bonn |
|---|---|---|---|---|---|---|---|
| 9 | 4 | 4 | 2 | 2 | >64 | >64 | >64 |
| 16 | 1 | 1 | 1 | 0,5 | >64 | >64 | >64 |
| 27 | 16 | 16 | 16 | 8 | >64 | >64 | >64 |

Die erfindungsgemäßen Verbindungen der allgemeinen Formeln (I), (Ia), (Ib), (Ic), (Id), (Ie) und (If) weisen bei geringer Toxizität ein breites antibakterielles Spektrum, speziell gegen gram-positive Bakterien sowie Mycobacterien,

Corynebacterien, Haemophilus influenzae und anaerobae Keime auf. Diese Eigenschaften ermöglichen ihre Verwendung als chemotherapeutische Wirkstoffe in der Human- und Tiermedizin.

Die erfindungsgemäßen Verbindungen sind gegen ein breites Spektrum von Mikroorganismen wirksam. Mit ihrer Hilfe können gram-positive Bakterien und bakterienähnliche Mikroorganismen, wie Mycoplasmen, bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch solche Erreger hervorgerufen werden.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30mg/kg Körpergewicht.

Die neuen Verbindungen können in den üblichen Konzentrationen und Zubereitungen zusammen mit dem Futter bzw. Lactamaseinhibitoren z.B. mit Penicillinen, die besonders penicillinasefest sind und Clavulansäure kombiniert werden. Eine solche Kombination wäre z.B. die mit Oxacillin oder Dicloxacillin.

Die erfindungsgemäßen Verbindungen können zum Zweck der Erweiterung des Wirkungsspektrums und um eine Wirkungssteigerung zu erreichen auch mit anderen Antibiotika kombiniert werden.

Anhang zum experimentellen Teil

Liste der verwendeten Laufmittelgemische zur Chromatographie:

| | |
|---|---|
| I | Dichormethan : Methanol |
| II | Toluol : Ethylacetat |
| III | Acetonitril : Wasser |
| I | V Ethylacetat |
| V | Petrolether : Ethylacetat |
| VI | Dichlormethan : Ethanol |
| VII | Toluol : Ethanol |
| VIII | Toluol: Aceton |

Abkürzungen:

| | |
|---|---|
| Z | Benzyloxycarbonyl |
| Boc | tert.Butyloxycarbonyl |
| DMF | Dimethylformamid Ph Phenyl |
| Me | Methyl |
| THF | Tetrahydrofuran |
| CDI | Carbonyldiimidazol |
| DCE | Dichlorethan |

**Ausgangsverbindungen**

**Beispiel I**

5-Benzyloxycarbonylamino-benzo[b]thiophen

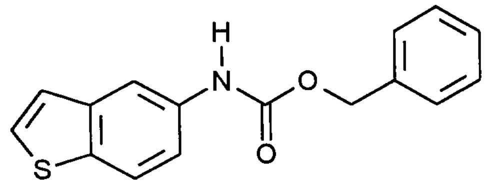

Zu einer Lösung von 40 g (268 mmol) 5-Amino-benzo[b]thiophen (Synthetic Communications, S. 959-964 (1991)) in 410 ml THF, 530 ml Wasser und 530 ml ges. $NaHCO_3$-Lösung werden langsam 44 ml (294 mmol) Chlorameisensäurebenzylester zugetropft. Man läßt über Nacht unter Rühren auf RT kommen, dampft das THF im Vakuum ab, extrahiert die wäßrige Phase 3 mal mit $CH_2Cl_2$, trocknet mit $Na_2SO_4$ und engt ein. Man nimmt in wenig $CH_2Cl_2$ auf, verdünnt mit Ether (ca. 200 ml) und versetzt unter Rühren langsam mit 1 l tiefsiedendem Petrolether. Man saugt den ausgefallenen Feststoff ab und trocknet bei 50°C über Nacht.
Ausbeute: 58,10 g (76,5%)
Smp.: 110°C
$^1$H-NMR ($D_6$-DMSO, TMS): 9,88 (s, 1H); 8,09 (d, J = 1 Hz, 1H); 7,9 (d, J = 9 Hz, 1H); 7,73 (d, J = 6 Hz, 1H); 7,3 - 7,5 (m, 7H); 5,2 (s, 2H).

In Analogie zu der Vorschrift des Beispiels I werden die in Tabelle I aufgeführten Verbindungen hergestellt:

**Tabelle I:**

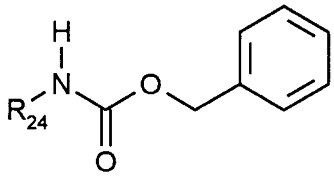

| Bsp.-Nr. | $R^{24}$ | Ausbeute (%) | Fp.: (°C) | $R_f$ Laufmittelgemisch (Verhältnis) |
|---|---|---|---|---|
| II | 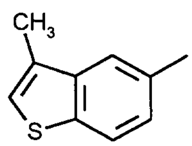 | 98 | 111 | 0,5 (1/1) V |
| III | 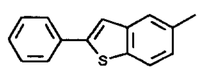 | 55 | - | 0,84 (100/5) I |
| IV | 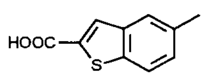 | 98 | 205 n.Z. | 0,3 (100/5) I |

| Bsp.-Nr. | $R^{24}$ | Ausbeute (%) | Fp.: (°C) | $R_f$ Laufmittelgemisch (Verhältnis) |
|---|---|---|---|---|
| V | 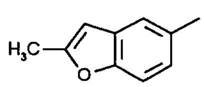 | 82 | 108°C | 0,68 (5/1) V |

**Beispiel VI**

5-Benzyloxycarbonylamino-benzo[b]thiophen-2-carbonsäure-tert.butylester

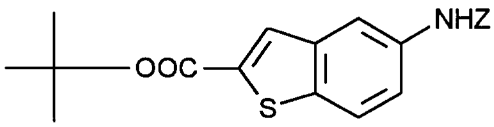

Eine Lösung von 25 g (76,4 mmol) der Verbindung aus Beispiel IV, 16,5 g (229 mmol) t-BuOH und 1,67 g (15,27 mmol) 4-Dimethylaminopyridin in 150 ml $CH_2Cl_2$ wird auf -10°C gekühlt, mit 16,10 g (84 mmol) 1-Ethyl-3-[3-(dimethylamino)propyl]carbodiimid Hydrochlorid versetzt und über Nacht unter Rühren auf RT gebracht. Die Lösung wird mit 200 ml $CH_2Cl_2$ verdünnt und je einmal mit verdünnter $H_2SO_4$, ges. $NaHCO_3$ und ges. NaCl-Lösung gewaschen, getrocknet und eingeengt. Das so erhaltene Rohprodukt wird an Kieselgel (PE / EE 1/1) chromatographiert.
Ausbeute: 7,5 g (26%)
$^1$H-NMR ($D_6$-DMSO, TMS): 10,0 (s, 1H); 8,2 (d, J = 2 Hz, 1H); 8,06 (s, 1H); 7,93 (d, J = 8 Hz, 1H); 7,58 (dd, J = 8 Hz, J = 2 Hz, 1H); 7,32 - 7,5 (m, 5H); 5,20 (s, 2H); 1,55 (s, 9H).

**Beispiel VII**

(5S)-(3-Methoxy-4-iodphenyl)-5-acetylaminomethyl-2-oxazolidinon

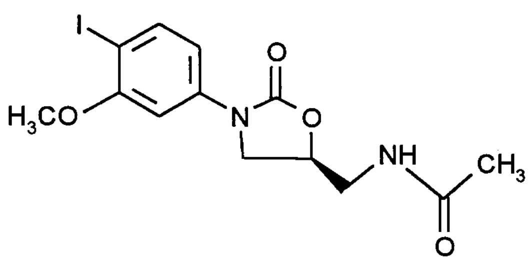

Zu einer Suspension von 264 mg (1,0 mmol) (5S)-(3-Methoxyphenyl)-5-acetylaminomethyl-2-oxazolidinon (J. Med. Chem. 35 (1992) 1156-1165), 250 mg (1,5 mmol) Silberacetat, 30 ml Dichlormethan und 20 ml Acetonitril werden 254 mg (1,0 mmol) Iod in 25 ml Dichlormethan zugegeben. Nach 16 h wird mit Wasser und Dichlormethan versetzt, die wäßrige Phase mit Dichlormethan extrahiert, die vereinigten organischen Phasen mit ges. NaCl-Lösung gewaschen, über $MgSO_4$ getrocknet und die Lösemittel im Vakuum abgezogen.
Ausbeute: 370 mg (95 %)
$^1$H-NMR ($D_6$-$CDCl_3$, TMS): 7,70 (d, 1H), 7,42 (d, 1H), 6,60 (dd, 1H), 6,25 (bt, 1H), 4,78 (m, 1H),4,05 (7, 1H), 3,89 (s, 3H), 3,50-3,85 (m, 3H), 2,00 (s, 3H).

**Beispiel VIII**

(5S)-(3-Hydroxy-4-iodphenyl)-5-acetylaminomethyl-2-oxazolidinon

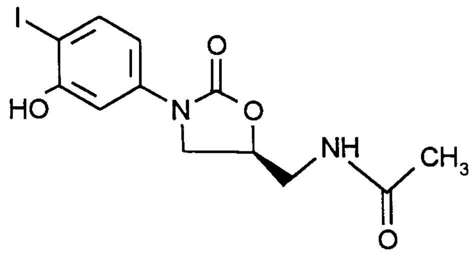

Zu einer Lösung aus 10.0 g (25,6 mmol) der Verbindung aus Beispiel VII in 100 ml Dichlormethan werden bei -78°C 77 ml (77 mmol) einer 1.0 M $BBr_3$-Lösung in Dichlormethan zugetropft. Man läßt auf Raumtemperatur kommen, rührt weitere 3h, kühlt auf -10°C und versetzt mit Wasser. Die Mischung wird mit Essigester versetzt, der ausgefallene Niederschlag abgesaugt und getrocknet.
Ausbeute: 8,0 g (83 %)
$R_f$ = 0,34 (Toluol/Aceton 1/1)
Smp.: 227-230°C
MS (DCI/$NH_3$): 377 ($M^+$ + H)
$[\alpha]^{20}_{D}$ = -18,76° (DMSO, c = 1,0)
$^1$H-NMR ($D_6$-DMSO, TMS): 10,5 (bs, 1H), 8,25 (bt, 1H), 7,62 (d, 1H), 7,30 (d, 1H), 6,68 (dd, 1H), 4,70 (m, 1H), 4,05 (t, 1H), 3,70 (dd, 1H), 3,40 (t, 1H), 1,80 (s, 3H).

**Herstellungsbeispiele**

**Beispiel 1**

(5R)-3-[5-Benzo[b]-thiophenyl]-5-hydroxymethyl-oxazolidin-2-on

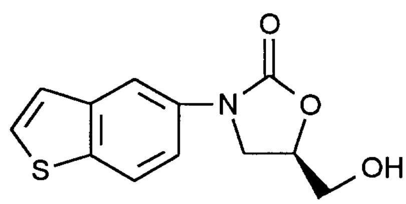

56,55 g (200 mmol) der Verbindung aus Beispiel I werden in 600 ml THF gelöst, mit 10 mg 1,10-Phenanthrolin-Hydrat versetzt und auf -70°C gekühlt. Nun werden langsam ca. 80 ml 2,5 N n-Butyllithium-Lösung in Hexan bis zum Farbumschlag nach rot zugetropft. Anschließend werden 28 ml (200 mmol) (R)-Glycidylbutyrat zugetropft. Man läßt auf RT kommen, versetzt mit gesättigter Ammoniumchlorid-Lösung, trennt die organische Phase ab und extrahiert die wäßrige Phase 2 mal mit Essigester. Die vereinigten organischen Phasen werden getrocknet ($Na_2SO_4$) und eingeengt. Der Rückstand wird in 1 l Ether verrührt, abgesaugt und getrocknet.
Ausbeute: 43,4 g (87,05%)
Smp.: 141°C
$[\alpha]^{20}_{D}$ = -58,9° (DMSO, c = 1,0)
MS (EI): 249 ($M^+$, 100%)
$^1$H-NMR ($D_6$-DMSO, TMS): 7,95 - 8,07 (m, 2H); 7,8 (d, J = 5 Hz, 1H); 7,7 (dd, J = 10 Hz, J = 2 Hz, 1H); 7,45 (d, J = 5 Hz, 1H); 5,23 (t, J = 6 Hz, 1H); 4,67 - 4,8 (m, 1H); 4,15 (t, J = 9 Hz, 1H); 3,92 (dd, J = 9 Hz, J = 5 Hz, 1H); 3,52 - 3,78 (m, 2H).

In Analogie zu der Vorschrift des Beispiels 1 werden die in Tabelle 1 aufgeführten Verbindungen hergestellt:

**Tabelle 1:**

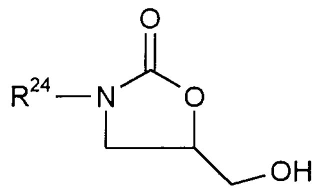

| Bsp.-Nr. | $R^{24}$ | Hergestellt aus Bsp.-Nr. | Ausbeute (% d.Th.) | Fp.: (°C) | $R_f$ Laufmittelgemisch (Verhältnis) |
|---|---|---|---|---|---|
| 2 | 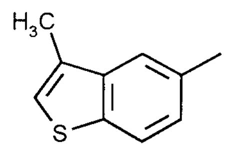 | II | 91 | 126 | 0,25 (3/7) V |
| 3 | 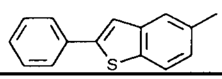 | III | 95 | 218 u.Z. | 0,5 (100/5) I |
| 4 | 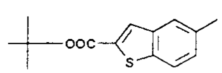 | IV | 73 | 130 | 0,1 (1/1) V |
| 5 | 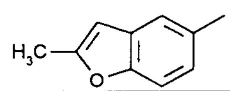 | V | 78 | 166 | 0,19 (1/1) V |

**Beispiel 6**

(5R)-3-[5-Benzo[b]-thiophenyl]-5-methansulfonylmethyl-oxazolidin-2-on

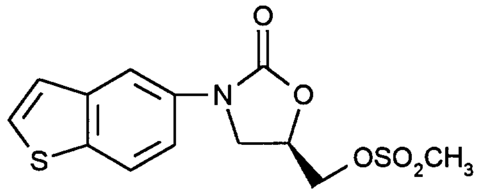

Eine Lösung von 42,4 g (170 mmol) der Verbindung aus Beispiel 1 und 59ml (425 mmol) Triethylamin in 2 l THF wird auf -10°C gekühlt und langsam mit 29 ml (374 mmol) Methansulfonsäurechlorid versetzt. Es wird 2 h bei -10°C nachgerührt, abgesaugt und der Rückstand 3 bis 4 mal mit $CH_2Cl_2$ nachgewaschen. Nachdem das Lösemittelgemisch am Rotationsverdampfer abgezogen wurde, wird der Rückstand in $CH_2Cl_2$ gelöst und je einmal mit ges. $NaHCO_3$, verd. $H_2SO_4$ und ges. NaCl-Lösung gewaschen. Nach Trocknen ($Na_2SO_4$) und Einengen erhält man das Produkt genügend rein für weitere Umsetzungen.
Ausbeute: 55,6 g (100%)

**Beispiel 7**

(5R)-3-[5-Benzo[b]thiophenyl]-5-azidomethyl-oxazolidin-2-on

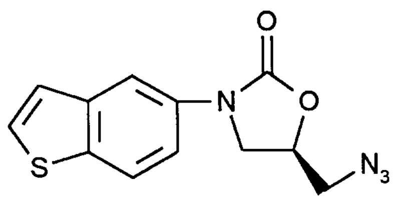

55 g (168 mmol) der Verbindung aus Beispiel 6 werden in 390 ml DMF gelöst und mit 12 g (185 mmol) Natriumazid versetzt. Die so erhaltene Reaktionsmischung wird 14 h bei 70°C gerührt. Man läßt auf RT abkühlen und kippt auf 3 l Eiswasser. Man saugt vom ausgefallenen Feststoff ab, wäscht mit Wasser und Petrolether nach und trocknet an der Luft.
Ausbeute: 43 g (93%)
Smp.: 108°C
$\alpha^{20}_D$ = -144,7° (DMSO, c = 1,0)
MS: 275 ($M^+$+1, 100%)
$^1$H-NMR ($D_6$-DMSO, TMS): 7,98 - 8,08 (m, 2H); 7,8 (d, J = 5 Hz, 1H); 7,68 (dd, J = 10 Hz, J = 2 Hz, 1H); 7,45 (d, J = 5 Hz, 1H); 4,85 - 5,0 (m, 1H); 4,21 (t, J = 9 Hz, 1H); 3,86 (dd, J = 9 Hz, J = 5 Hz, 1H); 3,75 (t, J = 6 Hz, 2H).

**Beispiel 8**

(5R)-3-[5-Benzo[b]-thiophenyl]-5-azidomethyl-oxazolidin-2-on Hydrochlorid

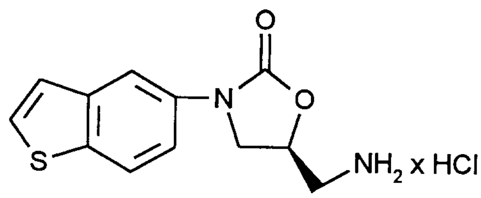

43 g (157 mmol) der Verbindung aus Beispiel 7 werden in 110 ml Ethylenglykoldimethylether gelöst und auf 50°C erwärmt. Man tropft langsam 22,2 ml (188 mmol) Trimethylphosphit zu (Gasentwicklung), erwärmt nach beendeter Zugabe auf 90°C und rührt 5 h bei 90°C nach. Nun tropft man 30 ml (180 mmol) 6 N HCl zu und rührt wieder 5 h bei 90°C nach. Man läßt auf RT abkühlen, saugt vom ausgefallenem Niederschlag ab, verrührt den Niederschlag in 1 l Ether für 1h, saugt wiederum vom Niederschlag ab und trocknet bei 50°C.
Ausbeute: 44 g (98%)
Smp.: 195°C
MS (DCI): 249 ($M^+$+1, 100%)

**Beispiel 9**

(5S)-3-[5-Benzo[b]thiophenyl]-5-acetylamino-methyl-oxazolidin-2-on

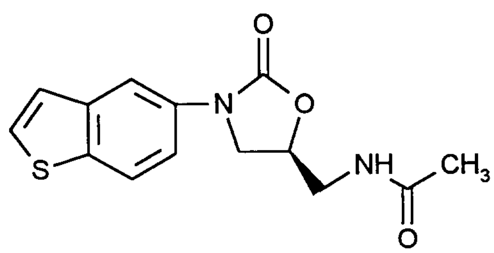

43 g (151 mmol) der Verbindung aus Beispiel 8 werden mit 320 ml $CH_2Cl_2$ und 54 ml (407 mmol) Triethylamin versetzt. Die so erhaltene Reaktionslösung wird unter Rühren auf 0°C gekühlt und langsam mit 16,5 ml (233 mmol) Essigsäurechlorid versetzt. Man rührt 5 h bei 0°C nach, verdünnt mit 400 ml Wasser und 300 ml $CH_2Cl_2$. Man trennt die organische Phase ab und wäscht diese je einmal mit verdünnter $H_2SO_4$, ges. $NaHCO_3$ und ges. NaCl-Lösung. Nach Trocknen ($Na_2SO_4$) und Einengen erhält man verunreinigtes Produkt, welches an Kieselgel (Methylenchlorid : Methanol 100:2) chromatographiert wird.
Ausbeute: 32,5 g (74,2%)
$R_f$ = 0,26 ($CH_2Cl_2$ / $CH_3OH$ = 100/5)
Smp.: 160°C
$^1$H-NMR ($D_6$-DMSO): 8,28 (t, J = 7 Hz, 1H); 7,95 - 8,05 (m, 2H); 7,8 (d, J = 5 Hz, 1H); 7,69 (dd, J = 10 Hz, J = 2 Hz, 1H); 7,45 (d, J = 5 Hz, 1H); 4,7 - 4,83 (m, 1H); 4,2 (t, J= 9 Hz, 1H); 3,85 (dd, J = 9 Hz, J = 5 Hz, 1H); 3,45 (t, J = 6 Hz, 2H); 1,85 (s, 3H).

In Analogie zu den Vorschriften der Beispiele 5 bis 9 werden die in der Tabelle 2 aufgeführten Verbindungen hergestellt:

**Tabelle 2:**

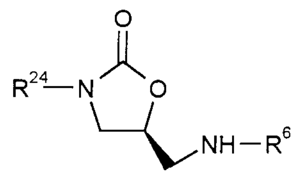

| Bsp.-Nr. | $R^{24}$ | $R^{6}$ | Ausbeute (% d.Th.) | Fp.: (°C) | hergestellt aus Bsp.-Nr. | $R_f$ Laufmittelgemisch (Verhältnis) |
|---|---|---|---|---|---|---|
| 10 | 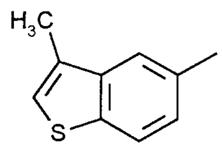 | 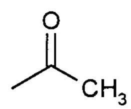 | 78 | 163 | 2 | |
| 11 | 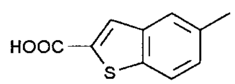 | 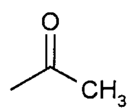 | 51 | | 4 | |
| 12 | 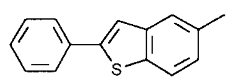 | 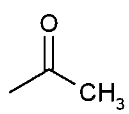 | 14 | 244 | 3 | |

| Bsp.-Nr. | $R^{24}$ | $R^6$ | Ausbeute (% d.Th.) | Fp.: (°C) | hergestellt aus Bsp.-Nr. | $R_f$ Laufmittelgemisch (Verhältnis) |
|---|---|---|---|---|---|---|
| 13 | 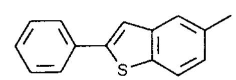 | 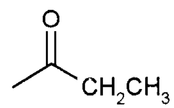 | 22 | 251 | 3 | |
| 14 | 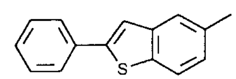 | 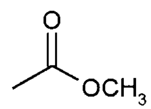 | 28 | 193 | 3 | |
| 15 | 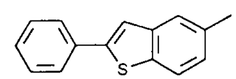 | 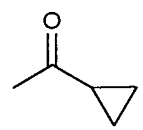 | 29 | 255 | 3 | |
| 16 | 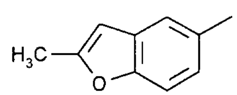 | 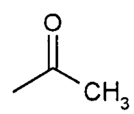 | 83 | 135 | 5 | 0,43 (10/1) VI |

**Beispiel 17**

(5S)-3-[5-(2-Brom-benzo[b]thiophenyl)]-5-acetylamino-methyl-oxazolidin-2-on

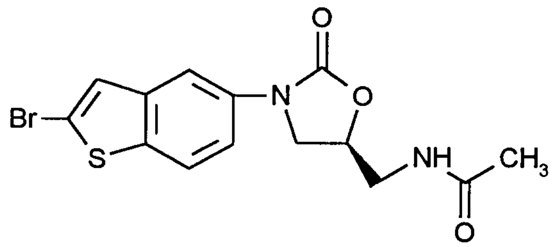

5 g (17,2 mmol) der Verbindung aus Beispiel 9 werden in 55 ml Chloroform und 35ml Acetonitril gelöst und auf 0°C gekühlt. Man tropft nun eine Lösung von 0,88 ml (17,2 mmol) Brom in 10 ml Chloroform langsam zu und rührt 72 h bei RT nach. Man saugt vom ausgefallenen Niederschlag ab und verrührt 16 h den Niederschlag mit 100 ml ges. $NaHCO_3$-Lösung. Man saugt vom Niederschlag ab, wäscht mit Wasser und Ether gut nach und trocknet bei 50°C.
Ausbeute: 4,01 g (63%)
Smp.: 132°C
$^1$H-NMR ($D_6$-DMSO): 8,28 (t, J = 7 Hz, 1H); 8,1 (d, J = 10 Hz, 1H); 8,07 (s, 1H); 7,92 (d, J = 2 Hz, 1H); 7,7 (dd, J = 10 Hz, J = 2 Hz, 1H); 4,72 - 4,82 (m, 1H); 4,26 (t, J = 9 Hz, 1H); 3,90 (dd, J = 9 Hz, J = 5Hz, 1H); 3,47 (t, J = 6 Hz, 2H); 1,83 (s, 3H).

In Analogie zu der Vorschrift des Beispiels 17 werden die in der Tabelle 3 aufgeführten Verbindungen hergestellt:

Tabelle 3:

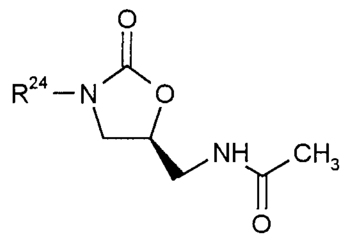

| Bsp.-Nr. | $R^{24}$ | Ausbeute (% d.Th.) | Fp.: (°C) | hergestellt aus Bsp.-Nr. | $R_f$ Laufmittelgemisch (Verhältnis) |
|---|---|---|---|---|---|
| 18 | 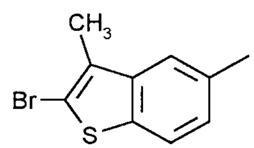 | 88 | 103 | 10 | 0,26 (100/5) I |
| 19 | 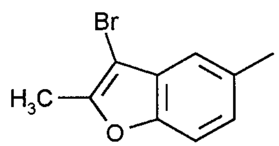 | 57 | 171 | 16 | 0,63 (1/1) VII |

**Beispiel 20**

(5S)-3-[5-(2-Phenyl-3-methyl-benzo[b]thiophenyl)]-5-acetylaminomethyl-oxazolidin-2-on

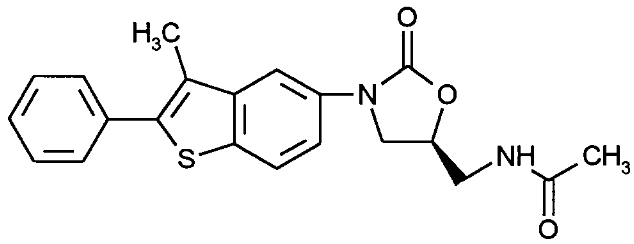

383 mg (1 mmol) der Verbindung aus Beispiel 18 und 159 mg (1,3 mmol) Phenylboronsäure werden in 8 ml THF gelöst, mit 34 mg (0,029 mmol) $Pd(P(C_6H_5)_3)_4$ versetzt und 1 h unter Rückfluß gekocht. Man gibt 1,4 ml 2 M $Na_2CO_3$ Lösung zu und kocht 16 h unter Rückfluß, kühlt auf RT ab, engt ein und chromatographiert an Kieselgel ($CH_2Cl_2$ / $CH_3OH$ 100:1).
Ausbeute: 336 mg (88%)
Smp.: 205°C
$R_f$ = 0,22 ($CH_2Cl_2$ / $CH_3OH$ 100/5)
MS: 380 ($M^+$, 100%)
$^1$H-NMR ($D_6$-DMSO, TMS): 8,28 (t, J = 7 Hz, 1H); 7,98 (d, J = 10 Hz, 1H); 7,88 (d, J = 2 Hz, 1H); 7,7 (dd, J = 2 Hz, J = 10 Hz, 1H); 7,4 - 7,62 (m, 5H); 4,7 - 4,85 (m, 1H); 4,28 (t, J = 9 Hz, 1H); 3,89 (dd, J= 9 Hz, J = 5 Hz, 1H); 3,45 (t, J = 6 Hz, 2H); 2,42 (s, 3H); 1,85 (s, 3H).

In Analogie zur Vorschrift des Beispiels 20 werden die in der Tabelle 4 aufgeführten Verbindungen hergestellt:

Tabelle 4:

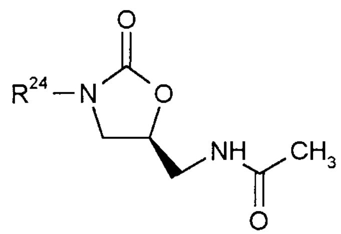

| Bsp.-Nr. | $R^{24}$ | Ausbeute (% d.Th.) | Fp.: (°C) | hergestellt aus Bsp.-Nr. | $R_f$ Laufmittelgemisch (Verhältnis) |
|---|---|---|---|---|---|
| 21 | 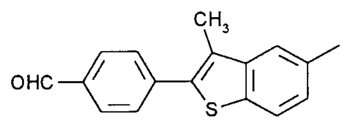 | 90 | 191 | 18 | 0,28 (100/5) I |
| 22 | 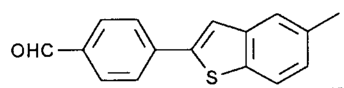 | 89 | 122 | 17 | 0,24 (100/5) I |
| 23 | 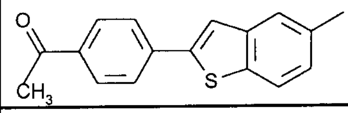 | 83 | 215 n.Z. | 17 | 0,24 (100/5) I |
| 24 | 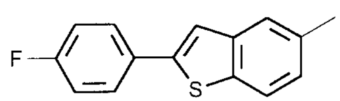 | 86 | 187 | 17 | 0,44 (100/5) I |
| 25 | 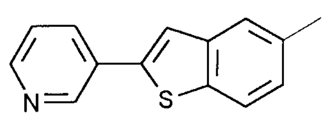 x HCl | 59 | 167 n.Z. | 17 | 0,14 (100/5) I |

**Beispiel 26**

(5S)-3-{5-[(2-Brom-1,1-dioxo-3-methyl)benzo[b]thiophenyl]}-5-acetylaminomethyl-oxazolidin-2-on

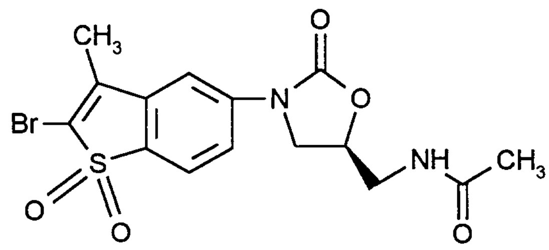

192 mg (0,5 mmol) der Verbindung aus Beispiel 18 und 344 mg (1,2 mmol) meta-Chlorperbenzoesäure werden in 2,5 ml $CH_2Cl_2$ gelöst und 48 h bei RT gerührt. Man verdünnt den Ansatz mit 20 ml Ether und 10 ml ges. $NaHCO_3$-Lösung und rührt die Mischung 1 h bei RT nach. Man saugt vom ausgefallenen Niederschlag ab, wäscht mit Ether nach und trocknet bei 50°C.

Ausbeute: 146 mg (71%)
$R_f$ = 0,17 ($CH_2Cl_2$ / $CH_3OH$ 100/3)
Smp.: 243°C
MS (FAB): 415 ($M^+$, 100%)
$^1$H-NMR ($D_6$-DMSO, TMS): 8,25 (t, J = 7 Hz, 1H); 8,0 (d, J = 10 Hz, 1H); 7,85 (d, J = 2 Hz, 1H); 7,73 (dd, J = 10 Hz, J = 2 Hz, 1H); 4,75 - 4,88 (m, 1H); 4,23 (t, J = 9 Hz, 1H); 3,84 (dd, J = 9 Hz, J = 5 Hz, 1H); 3,46 (t, J = 6 Hz, 2H); 2,28 (s, 3H); 1,83 (s, 3H).

In Analogie zur Vorschrift des Beispiels 26 werden die in der Tabelle 5 aufgeführten Verbindungen hergestellt:

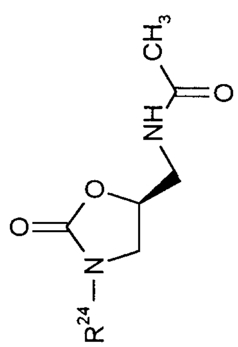

Tabelle 5:

| Bsp.-Nr. | $R^{24}$ | Ausbeute (% d.Th.) | Fp.: (°C) | hergestellt aus Bsp.-Nr. | $R_f$ Laufmittelgemisch (Verhältnis) |
|---|---|---|---|---|---|
| 27 | 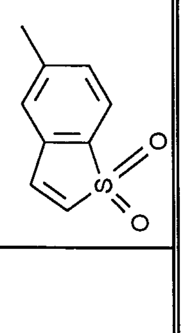 | 66 | 202 u.Z. | 9 | |

**Beispiel 28**

(5S)-(2-Hydroxymethyl-benzofuran-6-yl)-5-acetylaminomethyl-2-oxazolidinon

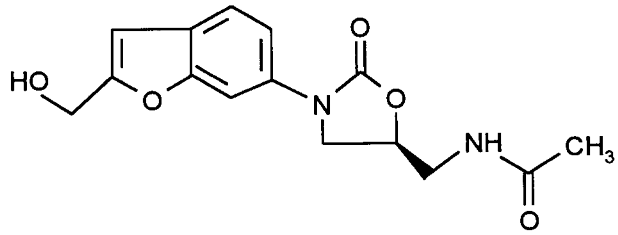

Eine Mischung aus 100 mg (0,27 mmol) der Verbindung aus Beispiel VIII, 55 mg (0,66 mmol) Natriumacetat, 30 mg (0,54 mmol) Propargylalkohol, 40 mg (0,05 mmol) Bis(triphenylphosphino)palladium(II)acetat, 20 mg (0,1 mmol) Kupferiodid in 2 ml DMF wird 14 h bei 60°C gerührt. Der Ansatz wird auf Eiswasser gegeben, mit Essigester extrahiert, die vereinigten organischen Phasen über $Na_2SO_4$ getrocknet, die Lösemittel im Vakuum abgezogen und der Rückstand durch Chromatographie (Toluol/Aceton) und Umkristallisation gereinigt.
Ausbeute: 40 mg (58 %)
$R_f$ = 0,25 (Toluol/Aceton 1/1)
Smp.: 142-144°C
MS (EI): 304 ($M^+$)
$^1$H-NMR ($D_6$-DMSO, TMS): 8,25 (bt, 1H), 7,75 (s, 1H), 7,57 (d, 1H), 7,40 (dd, 1H), 6,70 (s, 1H), 5,45 (t, 1H), 4,70 (m, 1H), 4,55 (d, 2H), 4,20 (t,1H), 3,75 (dd, 1H), 3,45 (m, 2H), 1,85 (s, 3H).

In Analogie zur Vorschrift des Beispiels 28 werden die in der Tabelle 6 aufgeführten Verbindungen hergestellt:

**Tabelle 6**

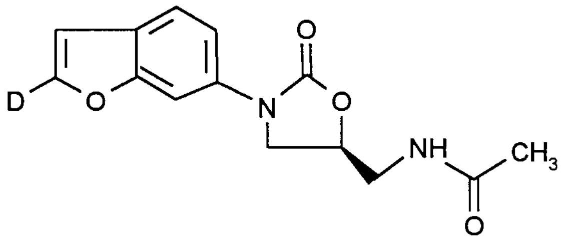

| Bsp.-Nr. | D | Ausbeute (% der Theorie) | $R_f$ (Laufmittelgemisch) Verhältnis | MS (DCI, $M^+$+H) |
|---|---|---|---|---|
| 29 | 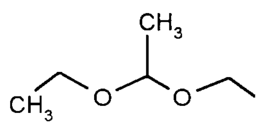 | 71 | 0,38 (VIII) 1:1 | 376 |
| 30 | 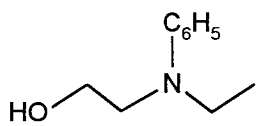 | 28 | 0,38 (VIII) 1:1 | 424 |
| 34 | 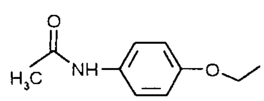 | 36 | 0,19 (VII) 1:1 | 438 |

**Patentansprüche**

1. Oxazolidinone der allgemeinen Formel (I)

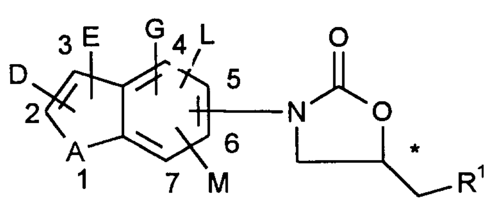

(I)

in welcher

$R^1$ für Azido, Hydroxy oder für eine Gruppe der Formel $-OR^2$, $O-SO_2R^3$ oder $-NR^4R^5$ steht, worin

$R^2$ geradkettiges oder verzweigtes Acyl mit bis zu 8 Kohlenstoffatomen oder eine Hydroxyschutzgruppe bedeutet,

$R^3$ geradkettiges oder verzweigtes Alkyl mit bis zu 4 Kohlenstoffatomen oder Phenyl bedeutet, das gegebenenfalls durch geradkettiges oder verzweigtes Alkyl mit bis zu 4 Kohlenstoffatomen substituiert ist,

$R^4$ und $R^5$ gleich oder verschieden sind und Cycloalkyl mit 3 bis 6 Kohlenstoffatomen, Wasserstoff, Phenyl oder geradkettiges oder verzweigtes Alkyl mit bis zu 8 Kohlenstoffatomen oder eine Aminoschutzgruppe bedeuten,

oder

$R^4$ oder $R^5$ eine Gruppe der Formel $-CO-R^6$ bedeutet,

worin

$R^6$ Cycloalkyl mit 3 bis 6 Kohlenstoffatomen, geradkettiges oder verzweigtes Alkyl oder Alkoxy mit jeweils bis zu 8 Kohlenstoffatomen , Phenyl oder Wasserstoff bedeutet,

A für ein Sauerstoff- oder Schwefelatom steht,

D, E, G, L und M gleich oder verschieden sind und für Wasserstoff, Carboxy, Halogen, Cyano, Mercapto, Formyl, Trifluormethyl, Nitro, geradkettiges oder verzweigtes Alkoxy, Alkoxycarbonyl, Alkylthio oder Acyl mit jeweils bis zu 6 Kohlenstoffatomen oder für geradkettiges oder verzweigtes Alkyl mit bis zu 6 Kohlenstoffatomen stehen, das seinerseits durch Hydroxy, durch geradkettiges oder verzweigtes Alkoxy oder Acyl mit bis zu 5 Kohlenstoffatomen, durch einen Rest der Formeln

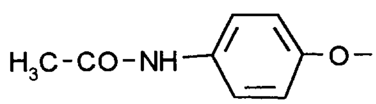
,

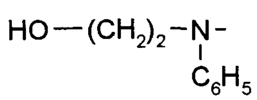
,

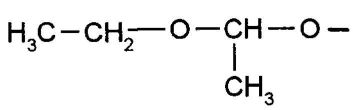

oder durch eine Gruppe der Formel $-NR^7R^8$ substituiert sein kann,

worin

$R^7$ und $R^8$ gleich oder verschieden sind und Wasserstoff, geradkettiges oder verzweigtes Alkyl mit bis zu 4 Kohlenstoffatomen oder Phenyl bedeuten,

oder gemeinsam mit dem Stickstoffatom einen 5- bis 6-gliedrigen, gesättigten Heterocyclus mit gegebenenfalls einem weiteren Heteroatom aus der Serie N, S und/oder O bilden, der seinerseits gegebenenfalls, auch an einem weiteren Stickstoffatom, durch geradkettiges oder verzweigtes Alkyl oder Acyl mit bis zu 3 Kohlenstoffatomen substituiert sein kann,

und/oder

gegebenenfalls für eine Gruppe der Formel $-NR^{7'}R^{8'}$ stehen,

worin

$R^{7'}$ und $R^{8'}$ gleich oder verschieden sind und die oben angegebene Bedeutung von $R^7$ und $R^8$ haben und mit dieser gleich oder verschieden sind,

und/oder

gegebenenfalls für ($C_2$-$C_8$)-Alkenylphenyl, Phenyl oder durch einen 5- oder 6-gliedrigen gesättigten oder ungesättigten Heterocyclus mit bis zu 3 Heteroatomen aus der Reihe S, N und/oder O stehen, die ihrerseits gegebenenfalls durch eine Gruppe der Formel -CO-$NR^9R^{10}$, $NR^{11}R^{12}$, -$NR^{13}$-$S(O)_2$-$R^{14}$, $R^{15}R^{16}N$-$SO_2$- oder $R^{17}$-$S(O)_a$- substituiert sind, worin

a — eine Zahl 0, 1 oder 2 bedeutet,

$R^9$, $R^{10}$, $R^{13}$, $R^{15}$ und $R^{16}$ — gleich oder verschieden sind und Wasserstoff, geradkettiges oder verzweigtes Alkyl mit bis zu 6 Kohlenstoffatomen oder Phenyl bedeuten,

$R^{11}$ und $R^{12}$ — gleich oder verschieden sind und die oben angegebene Bedeutung von $R^7$ und $R^8$ haben und mit dieser gleich oder verschieden sind,

$R^{14}$ und $R^{17}$ — gleich oder verschieden sind und die oben angegebene Bedeutung von $R^3$ haben und mit dieser gleich oder verschieden sind,

und/oder ihrerseits gegebenenfalls bis zu 2-fach gleich oder verschieden durch Carboxy, Halogen, Cyano, Mercapto, Formyl, Trifluormethyl, Nitro, Phenyl, geradkettiges oder verzweigtes Alkoxy, Alkoxycarbonyl, Alkylthio oder Acyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy, durch geradkettiges oder verzweigtes Alkoxy oder Acyl mit bis zu 5 Kohlenstoffatomen oder durch eine Gruppe der Formel -$NR^{18}R^{19}$ substituiert sein kann, worin

$R^{18}$ und $R^{19}$ — die oben angegebene Bedeutung von $R^7$ und $R^8$ haben und mit dieser gleich oder verschieden sind,

und deren Salze und S-Oxide.

**2.** Oxazolidinone nach Anspruch 1, worin

$R^1$ — für Azido, Hydroxy oder für eine Gruppe der Formel -$OR^2$, -$OSO_2R^3$ oder -$NR^4R^5$ steht, worin

$R^2$ — geradkettiges oder verzweigtes Acyl mit bis zu 6 Kohlenstoffatomen oder Benzyl bedeutet,

$R^3$ — geradkettiges oder verzweigtes Alkyl mit bis zu 3 Kohlenstoffatomen, Phenyl oder Toluoyl bedeutet,

$R^4$ und $R^5$ — gleich oder verschieden sind und Cyclopropyl, Cycylopentyl, Cyclohexyl, Wasserstoff, Phenyl oder geradkettiges oder verzweigtes Alkyl oder Alkoxy mit jeweils bis zu 6 Kohlenstoffatomen, tert. Butoxycarbonyl oder Benzyloxycarbonyl bedeuten, oder

$R^4$ oder $R^5$ — eine Gruppe der Formel -CO-$R^6$ bedeutet, worin

$R^6$ — Cyclopropyl, Cyclobutyl, Cyclopentyl, Cyclohexyl oder geradkettiges oder verzweigtes Alkyl oder Alkoxy mit jeweils bis zu 6 Kohlenstoffatomen, Phenyl oder Wasserstoff bedeutet,

A — für ein Sauerstoff- oder Schwefelatom steht,

D, E, G, L und M — gleich oder verschieden sind und für Wasserstoff, Carboxy, Fluor, Chlor, Brom, Jod, Cyano, Mercapto, Trifluormethyl, Formyl, Nitro, geradkettiges oder verzweigtes Alkoxy, Alkoxycarbonyl, Alkylthio oder Acyl mit jeweils bis zu 4 Kohlenstoffatomen oder für geradkettiges oder verzweigtes Alkyl mit bis zu 4 Kohlenstoffatomen stehen, das seinerseits durch Hydroxy, durch geradkettiges oder verzweigtes Alkoxy oder Acyl mit bis zu 4 Kohlenstoffatomen durch einen

Rest der Formeln

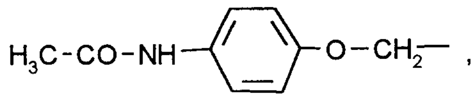

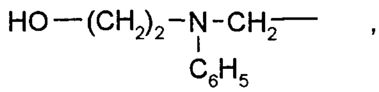

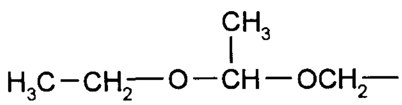

oder durch eine Gruppe der Formel $-NR^7R^8$ gegebenenfalls substituiert sein kann,
worin

| | |
|---|---|
| $R^7$ und $R^8$ | gleich oder verschieden sind und Wasserstoff, geradkettiges oder verzweigtes Alkyl mit bis zu 3 Kohlenstoffatomen oder Phenyl bedeuten, oder gemeinsam mit dem Stickstoffatom einen Morpholinyl-, Pyrrolidinyl-, Piperazinyl- oder Piperidylring bilden, die gegebenenfalls, auch über die freie N-Funktion, durch Methyl, Ethyl oder Acetyl substituiert sind, und/oder gegebenenfalls für eine Gruppe der Formel $-NR^{7'}R^{8'}$ stehen, worin |
| $R^{7'}$ und $R^{8'}$ | die oben angegebene Bedeutung von $R^7$ und $R^8$ haben und mit dieser gleich oder verschieden sind, und/oder gegebenenfalls für $(C_2$-$C_4)$-Alkenylphenyl, Phenyl, Pyridyl oder Thienyl stehen, die ihrerseits gegebenenfalls durch eine Gruppe der Formel $-CO-NR^9R^{10}$, $-NR^{11}R^{12}$, $-NR^{13}-SO_2-R^{14}$, $R^{15}R^{16}N-SO_2-$ oder $-R^{17}-S(O)_a$-substituiert sind, worin |
| a | eine Zahl 0, 1 oder 2 bedeutet, |
| $R^9$, $R^{10}$, $R^{13}$, $R^{15}$ und $R^{16}$ | gleich oder verschieden sind und Wasserstoff, geradkettiges oder verzweigtes Alkyl mit bis zu 4 Kohlenstoffatomen oder Phenyl bedeuten, |
| $R^{11}$ und $R^{12}$ | gleich oder verschieden sind und die oben angegebene Bedeutung von $R^7$ und $R^8$ haben und mit dieser gleich oder verschieden sind, |
| $R^{14}$ und $R^{17}$ | gleich oder verschieden sind und die oben angegebene Bedeutung von $R^3$ haben und mit dieser gleich oder verschieden sind, und/oder ihrerseits gegebenenfalls bis zu 2-fach gleich oder verschieden durch Carboxy, Fluor, Chlor, Brom, Jod, Cyano, Mercapto, Trifluormethyl, Formyl, Nitro, Phenyl, geradkettiges oder verzweigtes Alkoxy, Alkoxycarbonyl, Alkylthio oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, durch geradkettiges oder verzweigtes Alkoxy oder Acyl mit bis zu 4 Kohlenstoffatomen oder durch eine Gruppe der Formel $-NR^{18}R^{19}$ gegebenenfalls substituiert sein kann, worin |

| | |
|---|---|
| $R^{18}$ und $R^{19}$ | die oben angegebene Bedeutung von $R^7$ und $R^8$ haben und mit dieser gleich oder verschieden sind,<br>und deren Salze und S-Oxide. |

**3.** Oxazolidinone nach Anspruch 1, worin

| | |
|---|---|
| $R^1$ | für Azido, Hydroxy oder für eine Gruppe der Formel $-OR^2$, $-OSO_2R^3$ oder $-NR^4R^5$ steht, worin |
| $R^2$ | geradkettiges oder verzweigtes Acyl mit bis zu 6 Kohlenstoffatomen bedeutet, |
| $R^3$ | Methyl, Ethyl, Phenyl oder Toluoyl bedeutet, |
| $R^4$ und $R^5$ | gleich oder verschieden sind und<br>Cyclopropyl, Cyclopentyl, Cyclohexyl, Wasserstoff, Phenyl oder geradkettiges oder verzweigtes Alkyl mit bis zu 5 Kohlenstoffatomen bedeuten,<br>oder |
| $R^4$ oder $R^5$ | eine Gruppe der Formel $-CO-R^6$ bedeutet,<br>worin |
| $R^6$ | Cyclopropyl, Cyclopentyl, Cyclohexyl oder geradkettiges oder verzweigtes Alkyl oder Alkoxy mit jeweils bis zu 4 Kohlenstoffatomen, Wasserstoff oder Phenyl bedeutet, |
| A | für ein Sauerstoff- oder Schwefelatom steht, |
| D, E, G, L und M | gleich oder verschieden sind und<br>für Wasserstoff, Carboxy, Fluor, Chlor, Brom, Jod, Cyano, Formyl, Trifluormethyl, Nitro, geradkettiges oder verzweigtes Alkoxy, Alkoxycarbonyl oder Acyl mit jeweils bis zu 4 Kohlenstoffatomen oder für geradkettiges oder verzweigtes Alkyl mit bis zu 4 Kohlenstoffatomen stehen, das seinerseits durch Hydroxy, durch geradkettiges oder verzweigtes Alkoxy oder Acyl mit bis zu 4 Kohlenstoffatomen durch einen Rest der Formeln |

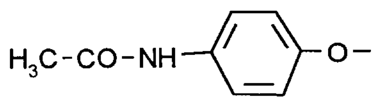 ,

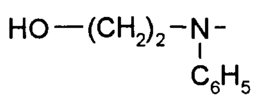 ,

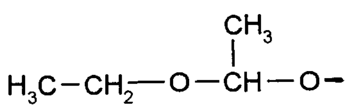

| | |
|---|---|
| | oder durch eine Gruppe der Formel $-NR^7R^8$ gegebenenfalls substituiert sein kann,<br>worin |
| $R^7$ und $R^8$ | gleich oder verschieden sind und Wasserstoff oder Methyl bedeuten,<br>oder gemeinsam mit dem Stickstoffatom einen Morpholinyl-, Pyrrolidinyl-, Piperazinyl- oder Piperidylring bilden, die gegebenenfalls, auch über die freie N-Funktion, durch Methyl, Ethyl oder Acetyl substituiert sind,<br>und/oder<br>gegebenenfalls für eine Gruppe der Formel $-NR^{7'}R^{8'}$ stehen,<br>worin |
| $R^{7'}$ und $R^{8'}$ | die oben angegebene Bedeutung von $R^7$ und $R^8$ haben und mit dieser gleich oder verschieden sind,<br>und/oder<br>gegebenfalls für 2-Phenylvinyl, Phenyl, Pyridyl oder Thienyl stehen, die ihrerseits gegebenenfalls durch eine Gruppe der Formel $-CO-NR^9R^{10}$ oder $-NR^{11}R^{12}$ substituiert sind,<br>worin |
| $R^9$ und $R^{10}$ | gleich oder verschieden sind und Wasserstoff oder Methyl bedeuten, |
| $R^{11}$ und $R^{12}$ | gleich oder verschieden sind und die oben angegebene Bedeutung von $R^7$ und $R^8$ haben und mit dieser gleich oder verschieden sind, |

und/oder ihrerseits gegebenenfalls bis zu 2-fach gleich oder verschieden durch Carboxy, Fluor, Chlor, Brom, Jod, Cyano, Formyl, Trifluormethyl, Nitro, Phenyl, geradkettiges oder verzweigtes Alkoxy, Alkoxycarbonyl oder Acyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy, durch geradkettiges oder verzweigtes Alkoxy oder Acyl mit bis zu 4 Kohlenstoffatomen oder durch eine Gruppe der Formel $-NR^{18}R^{19}$ gegebenenfalls substituiert sein kann,
worin

$R^{18}$ und $R^{19}$ die oben angegebene Bedeutung von $R^7$ und $R^8$ haben und mit dieser gleich oder verschieden sind,
und deren Salze und S-Oxide.

**4.** Oxazolidinone nach Anspruch 1, in welcher G, L und M für Wasserstoff stehen und der Oxazolidinonrest in den Positionen 5 oder 6 an den Phenylring angebunden ist.

**5.** Verwendung der Oxazolidinone nach den Ansprüchen 1 - 3 zur Herstellung von Arzneimitteln.

**6.** Arzneimittel, enthaltend Oxazolidinone gemäß den Ansprüchen 1 - 3.

Europäisches Patentamt

# EUROPÄISCHER RECHERCHENBERICHT

Nummer der Anmeldung
EP 95 11 0628

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A,D | EP-A-0 312 000 (E. I. DU PONT DE NEMOURS AND COMPANY)<br>* das ganze Dokument *<br>--- | 1,5 | C07D413/04<br>A61K31/42 |
| A,D | EP-A-0 311 090 (E. I. DU PONT DE NEMOURS AND COMPANY)<br>* das ganze Dokument *<br>--- | 1,5 | |
| A | WO-A-90 02744 (THE UPJOHN COMPANY)<br>* das ganze Dokument *<br>----- | 1,5 | |
| | | | RECHERCHIERTE SACHGEBIETE (Int.Cl.6) |
| | | | C07D |

Der vorliegende Recherchenbericht wurde für alle Patentansprüche erstellt

| Recherchenort | Abschlußdatum der Recherche | Prüfer |
|---|---|---|
| BERLIN | 29.November 1995 | Kyriakakou, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)